# EUROPEAN PATENT SPECIFICATION

(11) **EP 2 799 551 B1**
(45) Date of publication and mention of the grant of the patent: **04.07.2018**
(21) Application number: 12860693.6
(22) Date of filing: 20.12.2012
(51) Int. Cl.: C12N 15/10, G01N 33/50, G01N 33/58

(54) **METHOD FOR DETECTING COMPOUND-BINDING PROTEIN**
VERFAHREN ZUR ERKENNUNG EINES AN EINE VERBINDUNG BINDENDEN PROTEINS
PROCÉDÉ DE DÉTECTION D'UNE PROTÉINE SE LIANT À UN COMPOSÉ

(30) Priority: 20.12.2011 KR 20110137868
(43) Date of publication of application: 05.11.2014
(73) Proprietor: Korea Basic Science Institute, Daejeon 305-806 (KR)
(72) Inventor: LEE, Zee-Won, Daejeon 305-755 (KR); KIM, Soohyun, Daejeon 305-301 (KR); KIM, Seung II, Daejeon 305-755 (KR); HWANG, Jung Me, Daejeon 305-750 (KR); LEE, Kyung-Bok, Chungcheongbuk-do 363-783 (KR)
(74) Representative: Hoefer & Partner Patentanwälte mbB
(86) International application number: PCT/KR2012/011195
(87) International publication number: WO 2013/095014

(56) References cited:
- WO-A1-2009/049892
- WO-A2-03/029827
- WO-A2-2008/060483
- KR-A- 20070 006 569
- KR-A- 20070 048 660
- KR-B1- 100 948 767
- US-B1- 6 551 843

## Description

### [Technical Field]

The present description discloses a method for detecting an interaction between biomaterials, and more specifically, to a method for screening a prey which interacts with a bait, the method including (a) preparing a cell which expresses (i) a first construct comprising a translocation module, a first labeling material, and a first medium, and (ii) a third construct comprising a prey and a second labeling material; (b) introducing (iii) a second construct into the prepared cell, the second construct comprising a bait and a second medium binding with the first medium; (c) allowing the prey with the bait to interact each other; and (d) confirming the interaction between the prey and the bait by detecting intracellular distributions of the first construct and the third construct; along with a cell expressing the first construct and the third construct; and a screening kit comprising the cell and the second construct.

### [Background Art]

Growth, differentiation, migration, death and the like of cells are mediated by macromolecular interactions such as protein-protein or protein-nucleic acid interactions. Signals from outside of cells pass through receptors located on the cellular membrane and are transmitted to the nucleus of a cell through various biochemical reactions, where they express specific genes. This transfer of external signals into a cell is accomplished by protein interactions of several stages. For example, growth factors or cytokines bind to corresponding cell-surface receptors. This binding induces the receptors to cluster. The clustering of receptors by ligands induces clustering of the intracellular domains of the receptors, thereby causing interactions with signaling-related proteins. Through this signaling mechanism, intermediate proteins capable of transferring signals are produced by phosphorylation by protein kinases, dephosphorylation by protein phosphatases, or the like. As a result, the signals are transmitted to transcriptional activator proteins (Helden, C. H., (1995) Cell 80, 213-223). The activated transcriptional activator proteins bind to DNAs and interact with basal transcriptional regulator proteins such as RNA polymerases to activate specific genes. Such interactions enable transcription to occur specifically in specific tissues, in specific embryologic stages or in response to external stimulations. Abnormal modification, inhibition or acceleration of such interactions between specific proteins, which may be caused by intrusion of foreign matters, genetic modification of internal activator proteins or the like, may be the cause of a disorder. Accordingly, relevant researches have been continuously conducted because substances that can regulate the above said interactions may provide a way to treat the associated disorders.

The methods for analyzing the interactions of biomolecules, particularly the binding properties thereof, include traditional *in vitro* methods such as cross-linking, affinity chromatography, immunoprecipitation (IP), or the like. These methods require the production, isolation and purification of a protein and are disadvantageous in that an information different from the actual interaction may be obtained depending on the buffer condition in test tubes, a secondary modification of extracted proteins, or the like.

In order to make up for these drawbacks of the in vitro methods, in-cell methods such as yeast two-hybrid (Y2H), fluorescence resonance energy transfer (FRET) and bimolecular fluorescence complementation (Bi-FC) techniques have been developed. Each of these methods has advantages and disadvantages mentioned below.

Y2H is currently the most widely used technique along with immunoprecipitation. It is advantageous in that large-scale screening is possible using a gene library, but is disadvantageous in that investigation of membrane proteins or nuclear proteins such as transcriptase is difficult and there is a high probability of false positive. Besides, this method is inappropriate to find a substance capable of regulating protein-protein interactions. In the Y2H technique, the interaction between two proteins is detected based on the color change of colony to blue as X-gal compound in a medium is decomposed when β-galactosidase is expressed by the reporter gene. Since the screening technique of detecting the color change from blue back to white by a candidate substance is a negative screening, it is probable that a substance which has actually an inhibitory effect may be unnoticed. Further, since the detection itself is somewhat ambiguous, the technique is not suitable for general drug screening.

The FRET method provides good accuracy, but it is disadvantageous in that positioning of fluorescent proteins or fluorescent materials, which is required for the fluorescence resonance energy transfer to occur, is difficult, thereby having low rate of experimental success. The Bi-FC method is advantageous in that it is applicable for detecting membrane proteins or nuclear proteins. However, like the FRET method, it is disadvantageous in that relative positioning of proteins for complementary binding is difficult, thereby having low rate of success.

WO 03/029827 A2 discloses a 3-part hybrid system for detection of protein interactions in live mammalian cells and screening for compounds modulating such interactions. The three hybrids are a first heterologous conjugate comprising an anchor protein that specifically binds to an internal structure within the cell conjugated to an interactor protein of type A, a second heterologous conjugate comprising an interactor protein of type B conjugated to the first protein of interest, a third heterologous conjugate comprising a second protein of interest conjugated to a detectable group. When applying a dimerizer compound, interactor proteins A and B bind to each other and if the two proteins of interest interact, the distribution of the detectable group will mimic the distribution of the anchor protein. However, if there is no interaction, the distribution of the detectable group will mimic the distribution of the second protein of interest.

EP2187213 A1, which is a family member of KR 2008 0112354 A, discloses a method for detecting interactions of biomolecules. The disclosed method includes (a) preparing a cell comprising (i) a first construct comprising a bait, a first labeling material and a translocation module; and (ii) a second construct comprising a prey and a second labeling material; (b) detecting the distribution of the first construct and the second construct in the cell. The method is capable of detecting bindings and interactions occurring in a living cell in real time.

US2008/0242557 A1, which is a family member of KR 2007 0048660 A, discloses a method for detecting interactions between biomolecules, and also disclose a method for screening target molecules, both in vivo and in vitro. In this method, the first detecting material and the second detecting material are provided, wherein the first detecting material is bound to a localizer which is translocated by externally applied driving force, and the second detecting material is bound to a label. Thus, the complex of the first detecting material and the second detecting material can be detected reversibly by changing the strength of the externally applied driving force, and the target molecule can be screened effectively.

Therefore, various modified methods have been proposed to overcome the disadvantages of the above-described methods. However, there is a consistent need for an effective method for detecting the binding of biomaterials. Particularly, a detecting system enabling the detection of proteins interacting with target proteins and enabling a more efficient detection of regulator materials that inhibit or promote the interactions between two proteins is urgently needed.

The present inventors had developed a system in which a first construct comprising a protein kinase C, a first labeling material and a bait, and a second construct comprising a prey and a second labeling material are expressed in a cell, thereby confirming whether the expressed bait and prey interact each other, and obtained a patent for such a system (Korean Patent Registration No. 10-0948767). However, this system is usable only when both the bait and prey, of which the interaction is to be confirmed, are proteins generated in the cell, but not usable when the bait is not a protein expressed in the cell.

### [Detailed Description of the Invention]

### [Technical Problem]

While conducting researches on a more efficient search system which can detect a regulatory material capable of inhibiting or promoting an interaction between two proteins, the present inventors have developed a novel system capable of separating a bait from a translocation module and a first labeling material, and introducing the separated bait, and have completed the present invention.

Accordingly, an object of the present invention provides a method as defined in the claims for screening a prey which interacts with a bait, the method including
(a) preparing a cell which expresses (i) a first fusion construct comprising a translocation module, a first labeling material, and a first medium, and (ii) a third fusion construct comprising a prey and a second labeling material;
(b) introducing (iii) a second fusion construct into the prepared cell, the second construct comprising a bait and a second medium binding with the first medium;
(c) allowing the prey and the bait to interact each other; and
(d) inducing a translocation of the translocation module via an intrinsic signaling mechanism or by treating with a signaling material that generates an external signal; and (e) confirming the interaction between the prey and the bait by detecting intracellular distributions of the first construct and the third construct.

Another object of the present invention is to provide a cell as defined in the claims expressing (i) a first fusion construct comprising a translocation module, a first labeling material, and a first medium, and (ii) a third fusion construct comprising a prey and a second labeling material.

Still another object of the present invention is to provide a kit as defined in the claims for screening a prey which interacts with a bait, the kit comprising the cell and (iii) a second fusion construct comprising a second medium binding with a first medium and a bait.

### [Technical Solution]

In accordance with an aspect of the present invention, there is provided a method for screening a prey which interacts with a bait, the method as defined in the claims including:
(a) preparing a cell which expresses (i) a first fusion construct comprising a translocation module, a first labeling material, and a first medium, and (ii) a fusion third construct comprising a prey and a second labeling material;
(b) introducing (iii) a second fusion construct into the prepared cell, the second construct comprising a bait and a second medium binding with the first medium;
(c) allowing the prey and the bait to interact each other; and
(d) inducing a translocation of the translocation module via an intrinsic signaling mechanism or by treating with a signaling material that generates an external signal; and (e) confirming the interaction between the prey and the bait by detecting intracellular distributions of the first construct and the third construct.

In accordance with another aspect of the present invention, there is provided a cell as defined in the claims expressing: (i) a first fusion construct comprising a translocation module, a first labeling material, and a first medium; and (ii) a third fusion construct comprising a prey and a second labeling material.

In accordance with still another aspect of the present invention, there is provided a kit as defined in the claims for screening a prey which interacts with a bait, the kit comprising the cell; and (iii) a second fusion construct comprising a second medium binding with a first medium and a bait.

Hereafter, the present invention will be described in detail.

For the direct and real-time analysis of the interaction between biomaterials, the intracellular protein translocation occurred by an external stimulation or an intrinsic signaling mechanism is used in the present invention. In other words, the first fusion construct is designed to include a translocation module as defined in the claims which relocates by an external stimulation or an intrinsic signaling mechanism, a labeling material capable of tracing the translocation module, and a first medium enabling the binding of the translocation module and a bait. In addition, the second fusion construct is designed to include a bait which is one object of the interaction, and a second medium which binds with the first medium to bind the bait to the first fusion construct. In addition, the third fusion construct is designed to include a prey interacting with the bait, and a second labeling material capable of tracing the prey. Further, in an environment into which the second fusion construct is introduced, the first fusion construct and the third fusion construct are allowed to be expressed in a cell, the first fusion construct is allowed to bind with the second fusion construct, and the interaction between the bait and the prey is allowed to be analyzed directly and in real time in the cell.

Accordingly, the present invention provides a method for screening a prey which interacts with a bait, the method including
(a) preparing a cell which expresses (i) a first fusion construct comprising a translocation module, a first labeling material, and a first medium, and (ii) a third fusion construct comprising a prey and a second labeling material;
(b) introducing (iii) a second fusion construct into the prepared cell, the second construct comprising a bait and a second medium binding with the first medium;
(c) allowing the prey and the bait to interact each other; and
(d) inducing a translocation of the translocation module via an intrinsic signaling mechanism or by treating with a signaling material that generates an external signal; and
(e) confirming the interaction between the prey and the bait by detecting intracellular distributions of the first construct and the third construct.

As used herein, "a bait" (i.e., a material of
interest) and "a prey" (i.e., a target material) refer to materials that are subjected to an interaction, respectively. Each of the bait and the prey may be protein, polypeptide, small organic molecule, polysaccharide or polynucleotide, respectively. Preferably, they may be protein or polypeptide. Further, they may be a synthetic compound, chemical compound or a combination of two or more of them. For the purpose of detection or screening of an interaction, the bait may be a known material to a person conducting the detection or screening, while the prey may be an unknown material. But, without being limited thereto, the bait and the prey may be interchangeably included in the second construct or third construct.

As used herein, a first labeling material and a second labeling material refer to a material capable of generating a signal that can be distinctively detected by those skilled in the art. Examples include fluorescent materials, ligands, light-emitting materials, microparticles, redox molecules, radioactive isotopes. As for fluorescent materials, without being limited thereto, fluorescent protein, fluorescin, isothiocyanate, rhodamine, phycoerythrin, phycocyanin, alio phycocyanin, fluorescinisothiocyanate may be used. Among the above materials, as for fluorescent protein, those which are well known in the art may be used. Examples include GFP(Green Fluorescent Protein); EGFP(Enhanced Green Fluorescent Protein); RFP(Red Fluorescent Protein); mRFP(Monomeric Red Fluorescent Protein); DsRed(Discosoma sp. red fluorescent protein); CFP(Cyan Fluorescent Protein); CGFP(Cyan Green Fluorescent Protein); YFP(Yellow Fluorescent Protein); AzG(Azami Green), HcR(HcRed, Heteractis crispa red fluorescent protein), BFP(Blue Fluorescent Protein).

As for light-emitting materials, without being limited thereto, there are acridinium ester, luciferin, luciferase, or the like. As for microparticles, without being limited thereto, there are colloid gold, iron, colored latex or the like. As for redox molecules, without being limited thereto, there are ferrocene, ruthenium complex compounds, biologen, quinone, Ti ion, Cs ion, diimides, 1,4-benzoquinone, hydroquinone. As for radioactive isotopes, without being limited thereto, there are ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, ¹⁸⁶Re or the like. However, any one which could be used for detecting labelled materials can be utilized as well as the above material examples.

Preferably, a first labeling material and a second labeling material according to the present invention may be fluorescent proteins. More preferably, a first labeling material and a second labeling material of the present invention may be GFP; EGFP; RFP; mRFP; DsRed(Discosoma sp. red fluorescent protein); CFP(Cyan Fluorescent Protein); CGFP(Cyan Green Fluorescent Protein); YFP(Yellow Fluorescent Protein); AzG(Azami Green), HcR(HcRed, Heteractis crispa red fluorescent protein), or BFP(Blue Fluorescent Protein).

In the present invention, the translocation module as defined in the claims serves to move the first fusion construct to a specific region in a cell. The translocation to the specific region may be induced by an external signal or induced intrinsically. The specific region in a cell refers to an intracellular structure which is separate, discreet and identifiable. Preferably, the specific region may be membranous structures such as cell membrane, plasma membrane, and nuclear membrane; organelles such as endoplasmic reticulum, Golgi apparatus, mitochondria, and lysosome; or other specific regions in a cell.

As used herein, the translocation module may be different depending on the particular specific region in a cell. The translocation module is protein kinase C (PKC), including classical PKCs (cPKCs; PKC-alpha, PKC-beta and PKC-gamma), novel PKCs (nPKCs; PKC-delta, PKC-epsilon, PKC-eta and PKC-theta), atypical PKCs (aPKCs; PKC-zeta and PKC-lambda/iota) and their variants, as known in the art and defined in SEQ ID NOs: 1, 3, 5, and 7.

All of them have the CI domain in common. When diacylglycerol (DAG) or phorbol ester (TPA or PMA) binds at the CI domain, they are induced to move toward the cell membrane. Preferably, a variant of PKC may be used as the translocation module of the present invention. More preferably, the variant may be one from which the internal phosphorylation active site of PKC is removed in order to minimize interference caused by an internal signaling mechanism. The translocation module of the present invention has an amino acid sequence of SEQ ID NO: 1 (PRKCD), SEQ ID NO: 3 (TMA), SEQ ID NO: 5 (TMB) or SEQ ID NO: 7(TMD), or a nucleotide sequence of SEQ ID NO: 2 (PRKCD), SEQ ID NO: 4 (TMA), SEQ ID NO: 6 (TMB) or SEQ ID NO: 8 (TMD).

The media according to the present invention are used for the intracellular binding of the first construct and the second construct, and thus the binding specificity and the binding strength therebetween are high. The first medium can be directly produced through gene translation and gene expression in a cell. The second medium is a material which has a small molecular weight and thus can easily penetrate into a cell.

As for the media of the present invention, the first medium may be preferably streptavidin and the second medium may be preferably biotin. Alternatively, when the first medium is dihydrofolate reductase (DHFR), the second medium may be methotrexate (MTX), or when the first medium is a histidine polymer (His-tag), the second medium may be nickle-nitrilotriacetic acid (Ni-NTA) .

Meanwhile, the translocation of the first construct and the third construct may be detected in a cell, and may be preferably detected in the plasma membrane. In the absence of the translocation signal, the first construct and the third construct may be detected in a cell, and preferably may be detected in the cytosol. The translocation of the third construct to the plasma membrane is conducted by an interaction between the bait of the second construct and the prey of the third construct. Therefore, the translocation of the third construct to the plasma membrane indicates the interaction between the bait of the second construct and the prey of the third construct.

Thus, the method of the present invention enables real-time monitoring of direct binding or complex binding of biomolecules in a living cell through imaging, and provides the following advantages over existing techniques:
1) All bindings occurring in a living cell can be analyzed.
2) Accurate analysis is possible because the positional change in a cell is monitored, differently from other methods where the whole cell is monitored.
3) Unlike *in vitro* methods, no influence from external environment occurs, because binding occurring in a living cell is monitored.
4) The binding of a bait and a prey can be monitored in real time.
5) The complex binding of a bait with multiple preys can be monitored.
6) Screening of binding of a prey to an unknown biomaterial is possible by using a mass marker library for the prey.
7) A high-throughput system can be implemented in association with a high-content screening (HCS) system.
8) Binding characteristics can be analyzed for different signaling pathways by changing the kind of external stimulation.
9) Relative quantification of the bait and the prey is possible by labeling both the first construct and second construct with labeling materials. False positive or false negative responses can be significantly reduced because experimental errors related to the translocation of the prey in response to external stimulation or via intrinsic signaling mechanisms can be simultaneously verified.
10) The interaction between a material which is not directly produced through gene translation and gene expression in a cell and a material which is expressed in a cell can be measured.

As described above, the translocation module of the present invention may move to a specific region in a cell by an external signal. Accordingly, the inventive method for screening a prey which interacts with a bait may further include performing treatment with a signaling material. That is, the method of the present invention may include
(a) preparing a cell which expresses (i) a first fusion construct comprising a translocation module, a first labeling material, and a first medium, and (ii) a fusion third construct comprising a prey and a second labeling material;
(b) introducing (iii) a second fusion construct into the prepared cell, the second construct comprising a bait and a second medium binding with the first medium;
(c) allowing the prey and the bait to interact each other; and
(d) inducing a translocation of the translocation module by treating with a signaling material that generates an external signal; and
(e) confirming the interaction between the prey and the bait by detecting intracellular distributions of the first construct and the third construct.

The signaling material refers to a material which generates an external signal inducing the translocation of the translocation module. As PKC is used as the translocation module, the signaling material may be phorbol-12-myristate 13-acetate (PMA; phorbol ester), 12-O-tetradecanoylphorbol-13-acetate (TPA), phorbol-12,13-dibutyrate (PDBu), adenosine triphosphate (ATP), tridecanoic acid, arachidonic acid, linoleic acid, DiC8, 130C937, PKC activation-related growth factors or other PKC activating materials. Preferably, the signaling material may be phorbol-12-myristate 13-acetate.

PMA may be treated at a concentration of preferably 50 nM to 5 µM, more preferably 1 µM. If the PMA concentration is below 50 nM, translocation of the PKC translocation module may be insufficient. Otherwise, if it exceeds 5 uM, excessive treatment of the chemical may lead to such undesired phenomena as cell death, signaling interference or the like.

In addition, the present invention provides a cell as defined in the claims expressing: (i) a first fusion construct comprising a translocation module, a first labeling material, and a first medium; and (ii) a third fusion construct comprising a prey and a second labeling material. The translocation module, the first labeling material, the first medium, the prey, and the second labeling material are as described above.

As used herein, the cell may be a cell of an animal, a plant, an yeast or a bacteria. Preferably, except for bacteria, the cell may be a cell which is capable of effectively accepting the first construct introduced from outside and has well-defined boundaries of cytoplasm, nucleus and organelles. More preferably, the cell may be CHO-k1(ATCC CCL-61, *Cricetulus griseus,* hamster, Chinese), HEK293(ATCC CRL-1573, *Homo sapiens,* human), HeLa(ATCC CCL-2, *Homo sapiens,* human), SH-SY5Y(ATCC CRL-2266, *Homo sapiens,* human), Swiss 3T3(ATCC CCL-92, *Mus musculus,* mouse), 3T3-L1(ATCC CL-173, *Mus musculus,* mouse), NIH/3T3(ATCC CRL-1658, *Mus musculus,* mouse), L-929(ATCC CCL-1, *Mus musculus,* mouse), Rat2(ATCC CRL-1764, *Rattus norvegicus,* rat), RBL-2H3(ATCC CRL-2256, *Rattus norvegicus,* rat), or MDCK(ATCC CCL-34, *Canis familiaris*). In addition, the cell may be stem cells, cells extracted from various tissues and the artificially-prepared mimic cell membrane structure.

In the present invention, the cell comprising the first construct and third construct may be prepared by molecular biological techniques known in the art. Although not limited thereto, expression vectors capable of expressing each of the first construct and the third construct, respectively, or an expression vector capable of expressing both the first construct and third construct may be introduced into a cell, so that the first construct and third construct are preferably expressed by the expression vector(s).

To this end, as for the first construct, an expression vector comprising a promoter and a nucleotide encoding a first medium, a first labeling material and a translocation module, which is operably linked thereto, may be constructed. As for the third construct, an expression vector comprising a promoter and a nucleotide encoding a prey and a second labeling material, which is operably linked thereto, may be constructed. The two expression vectors may be simultaneously or sequentially introduced into a single cell, so that the first construct and third construct are expressed by the expression vectors. The sequence of the first medium, the first labeling material and the translocation module in the nucleotide is not important, as long as the function of the present invention is exerted. The same is true for the nucleotide encoding the prey and the second labeling material.

As used herein, the "promoter" means a DNA sequence regulating the expression of nucleic acid sequence operably linked to the promoter in a specific host cell, and the term "operably linked" means that one nucleic acid fragment is linked to other nucleic acid fragment so that the function or expression thereof is affected by the other nucleic acid fragment. Additionally, the promoter may include a operator sequence for controlling transcription, a sequence encoding a suitable mRNA ribosome-binding site, and a sequence controlling the termination of transcription and translation.

The introduction of an expression vector to a cell may be performed by the transfection methods which are well known in the art, for example, calcium phosphate method, calcium chloride method, rubidium chloride method, microprojectile bombardment, electroporation, particle gun bombardment, Silicon carbide whiskers, sonication, PEG-mediated fusion, microinjection, liposome-mediated method, magnetic nanoparticle-mediated method and the like.

Meanwhile, as for the cell according to the present invention, when the second construct comprising the bait and the second medium is introduced into the cell of the present invention, the second construct binds with the first construct expressed in the cell, and the second construct and the third construct bind to each other depending on whether the bait and the prey interact with each other. Therefore, the interaction between the bait and the prey can be confirmed by detecting the first labeling material and the second labeling material included in the first construct and the third construct. In this way, the interaction between a material which is not directly produced through gene translation and gene expression in the cell and a material expressed in the cell can be measured.

Meanwhile, general recombinant DNA and molecular cloning techniques of the present invention are well known in the art and they are well described in the following references (Sambrook, J., Fritsch, E. F. and Maniatis, T., Molecular Cloning: A Laboratory Manual, 2nd ed., Cold Spring Harbor Laboratory: Cold Spring Harbor, NY (1989); Silhavy, T. J., Bennan, M. L. and Enquist, L. W., Experiments with Gene Fusions, Cold Spring Harbor Laboratory: Cold Spring Harbor, NY (1984); and Ausubel, F. M. et al., Current Protocols in Molecular Biology, published by Greene Publishing Assoc. and Wiley-Interscience (1987)).

Further, the present invention provides a kit as defined in the claims for screening a prey interacting with a bait, the kit comprising a cell comprising a first fusion construct and a third fusion construct according to the present invention; and (iii) a second fusion construct comprising a second medium binding with a first medium and a bait.

The kit of the present invention may preferably comprise a signal material for enabling the interaction between the prey and the bait. The signal material is as described above, and may be preferably phorbol 12-myristate 13-acetate (PMA, phorbol ester).

Preferably, as for the media of the kit of the present invention, the first medium may be streptavidin and the second medium may be biotin. Alternatively, the first medium may be dihydrofolate reductase (DHFR) and the second medium may be methotrexate (MTX).

The kit of the present invention may further comprise a tool and/or a reagent, which are used to detect a labeling material and are known in the art, in addition to the cell comprising the first construct and the third construct of the present invention and (iii) the second construct comprising the second medium binding with the first medium and the bait. The kit of the present invention may further comprise a tube for mixing each components, a well plate, an instruction manual describing how to use, or the like, if necessary.

Experimental procedures, reagents, and reaction conditions that may be used in the methods of the present invention may be those commonly known in the art and will be obvious to those skilled in the art.

### [Advantageous Effects]

Accordingly, the present invention provides a method as defined in the claims for screening a prey which interacts with a bait, the method including (a) preparing a cell which expresses (i) a first fusion construct comprising a translocation module, a first labeling material, and a first medium, and (ii) a third fusion construct comprising a prey and a second labeling material; (b) introducing (iii) a second fusion construct into the prepared cell, the second construct comprising a bait and a second medium binding with the first medium; (c) allowing the prey and the bait to interact each other; and (d) inducing a translocation of the translocation module via an intrinsic signaling mechanism or by treating with a signaling material that generates an external signal; and (e) confirming the interaction between the prey and the bait by detecting intracellular distributions of the first construct and the third construct. Further, the present invention provides a cell as defined in the claims expressing the first construct and the third construct. Still further, the present invention provides a screening kit as defined in the claims comprising the cell and the second construct. The methods of the present invention can overcome disadvantages including inaccuracy and complexity of the existing techniques for biomaterial interaction detection, and can measure the interaction between a material which is not directly produced through gene translation and gene expression in the cell and a material expressed in a cell. Thus, the methods of the present invention are effective in detecting interactions of a broad range of biomaterials.

### [Brief Description of the Drawings]

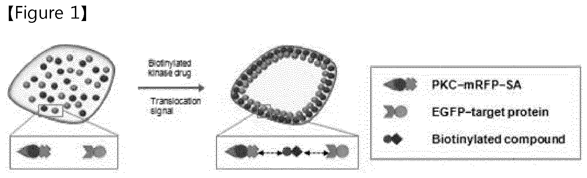

FIG. 1 shows a basic conceptual view showing the detection of binding according to the present invention.

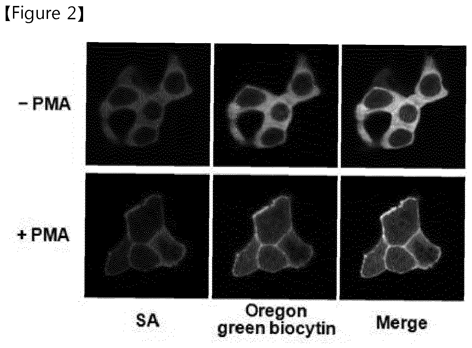

FIG. 2 illustrates results confirming whether a second construct can be detected by a first construct (-PMA: before treatment with signal material (PMA), +PMA: after treatment with signal material, SA: detection images of a first construct comprising streptavidin and RFP, Oregon green biocytin: detection images of a third construct comprising GFP, Merge: merging of the detection images of SA and detection images of Oregon green biocytin).

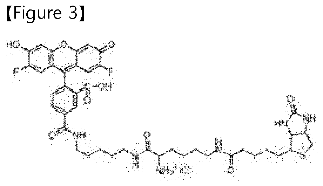

FIG. 3 shows a chemical structure of a compound-biotin (biocytin) used in FIG. 2.

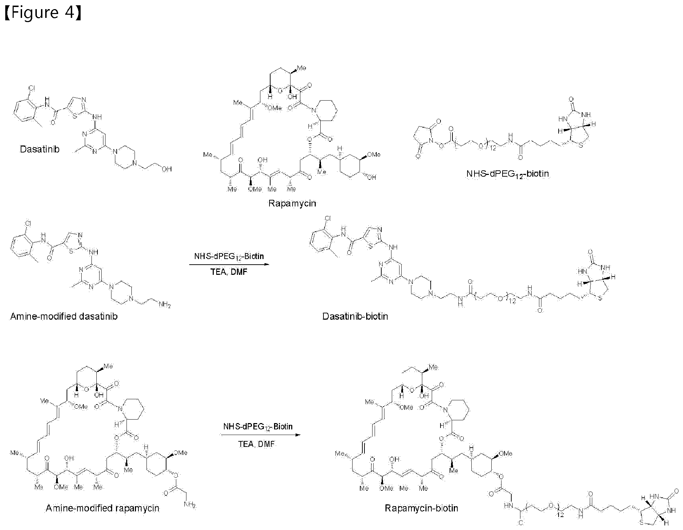

FIG. 4 shows synthetic mechanisms and chemical structures of dasatinib-biotin and rapamycin-biotin, which are synthesized for the verification of the present invention.

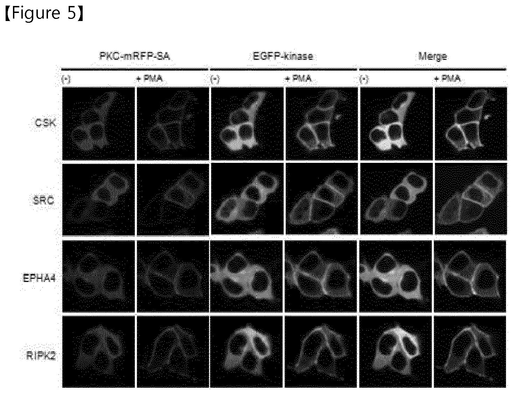

FIG. 5 illustrates results of detecting the binding with respect to CSK, SRC, EPHA4, and RIPK2 proteins, the binding being detected by dasatinib-biotin used as a second medium (CSK: C-src tyrosine kinase, SRC: Proto-oncogene tyrosine-protein kinase Src, EPHA4: ephrin type-A receptor 4, RIPK2: Receptor-interacting serine-threonine kinase 2, (-): before treatment with signal material (PMA), +PMA: after treatment with signal material, PKC-mRFP-SA: detection images of a first construct comprising streptavidin and RFP, EGFP-kinase: detection images of a third construct comprising GFP, Merge: merging of the detection images of PKC-mRFP-SA and detection images of EGFP-kinase)

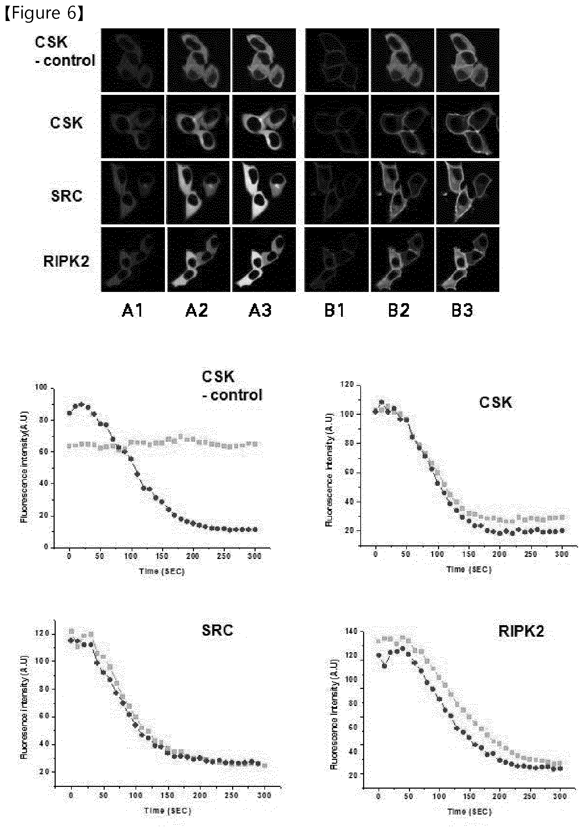

FIG. 6 illustrates results of detecting the binding with respect to CSK, SRC, and RIPK2 and charting the fluorescence change (reduction) in the cytosol (CSK: C-src tyrosine kinase, SRC: Proto-oncogene tyrosine-protein kinase Src, EPHA4: ephrin type-A receptor 4, RIPK2: Receptor-interacting serine-threonine kinase 2, A1: detection images of a first construct comprising RFP before treatment with PMA, A2: detection images of a third construct comprising GFP before treatment with PMA, A3: merging of the detection images of A1 and A2, B1: detection images of a first construct comprising RFP after treatment with PMA, B2: detection images of a third construct comprising GFP after treatment with PMA, B3: merging of the detection images of B1 and B2).

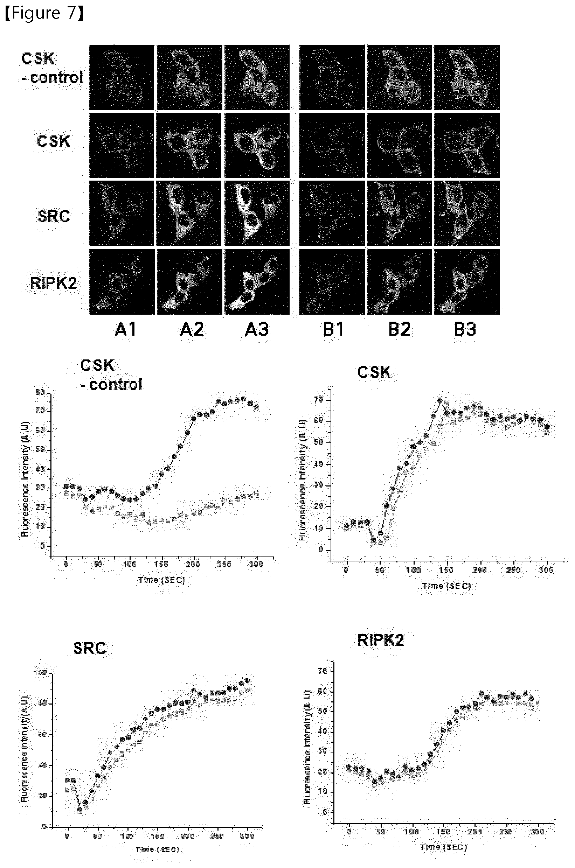

FIG. 7 illustrates results of detecting the binding with respect to CSK, SRC, and RIPK2 and charting the fluorescence change (increase) in the cytosol (CSK: C-src tyrosine kinase, SRC: Proto-oncogene tyrosine-protein kinase Src, EPHA4: ephrin type-A receptor 4, RIPK2: Receptor-interacting serine-threonine kinase 2, A1: detection images of a first construct comprising RFP before treatment with PMA, A2: detection images of a third construct comprising GFP before treatment with PMA, A3: merging of the detection images of A1 and A2, B1: detection images of a first construct comprising RFP after treatment with PMA, B2: detection images of a third construct comprising GFP after treatment with PMA, B3: merging of the detection images of B1 and B2).

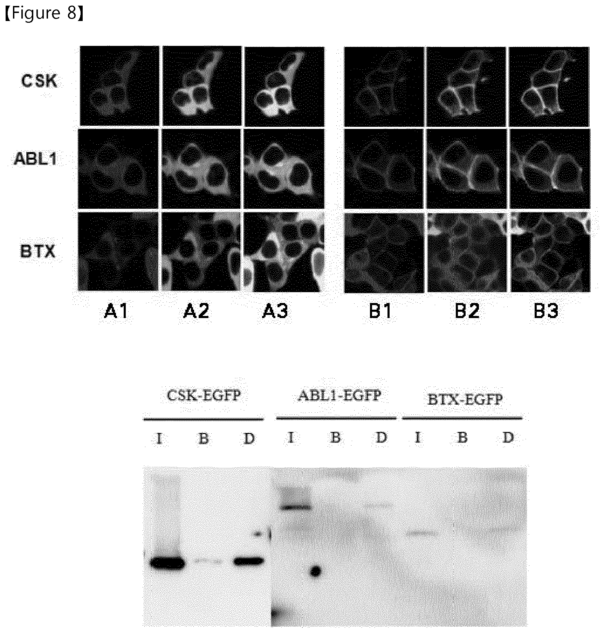

FIG. 8 illustrates detection images of the binding with respect to ABL kinase family binding with dasatinib and results of immunoprecipitation (IP) for verifying the binding (CSK: C-src tyrosine kinase, ABL1: V-abl Abelson murine leukemia viral oncogene homolog 1, A1: detection images of a first construct comprising RFP before treatment with PMA, A2: detection images of a third construct comprising GFP before treatment with PMA, A3: merging of the detection images of A1 and A2, B1: detection images of a first construct comprising RFP after treatment with PMA, B2: detection images of a third construct comprising GFP after treatment with PMA, B3: merging of the detection images of B1 and B2).

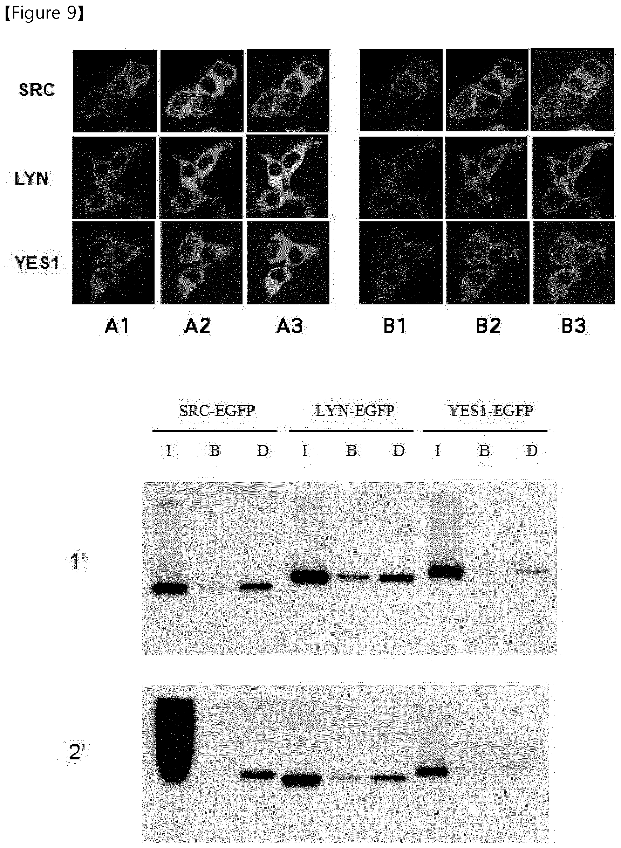

FIG. 9 illustrates detection images of the binding with respect to SRC kinase family binding with dasatinib and results of immunoprecipitation (IP) for verifying the binding (SRC: Proto-oncogene tyrosine-protein kinase Src, LYN: Tyrosine-protein kinase Lyn, YES1: Proto-oncogene tyrosine-protein kinase Yes, A1: detection images of a first construct comprising RFP before treatment with PMA, A2: detection images of a third construct comprising GFP before treatment with PMA, A3: merging of the detection images of A1 and A2, B1: detection images of a first construct comprising RFP after treatment with PMA, B2: detection images of a third construct comprising GFP after treatment with PMA, B3: merging of the detection images of B1 and B2).

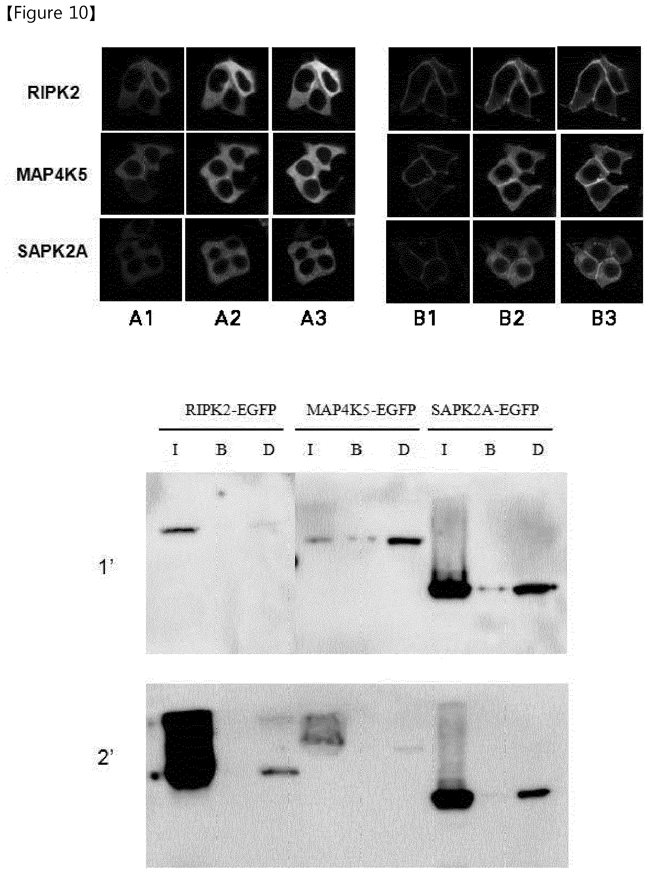

FIG. 10 illustrates detection images of the binding with respect to RIPK, MAP4K5, and SAPK2A proteins, which bind with dasatinib and of which functional correlation is not known, and results of immunoprecipitation (IP) for verifying the binding (RIPK2: Receptor-interacting serine-threonine kinase 2, MAP4K5: Mitogen-activated protein kinase kinase kinase kinase 5, SAPK2A: Stress Activated Protein Kinase 2a, A1: detection images of a first construct comprising RFP before treatment with PMA, A2: detection images of a third construct comprising GFP before treatment with PMA, A3: merging of the detection images of A1 and A2, B1: detection images of a first construct comprising RFP after treatment with PMA, B2: detection images of a third construct comprising GFP after treatment with PMA, B3: merging of the detection images of B1 and B2).

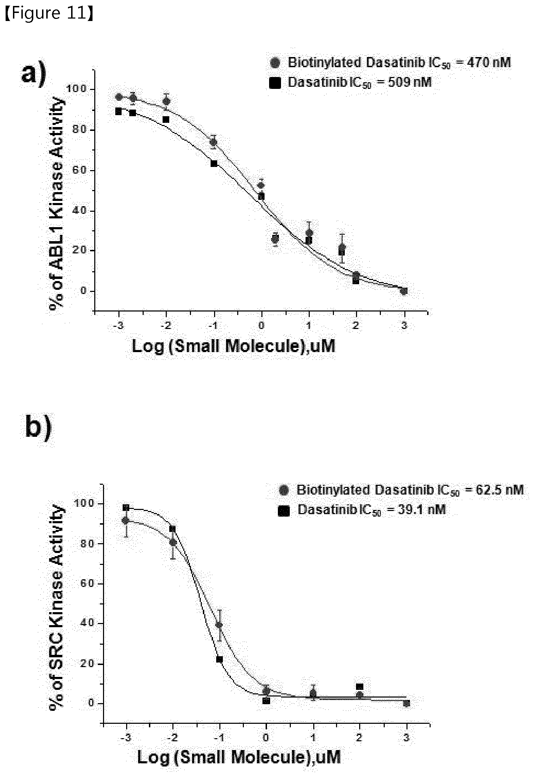

FIG. 11 illustrates results of verifying that phosphorylation activity of original unbiotinylated dasatinib was similar to that of biotinylated dasatinib.

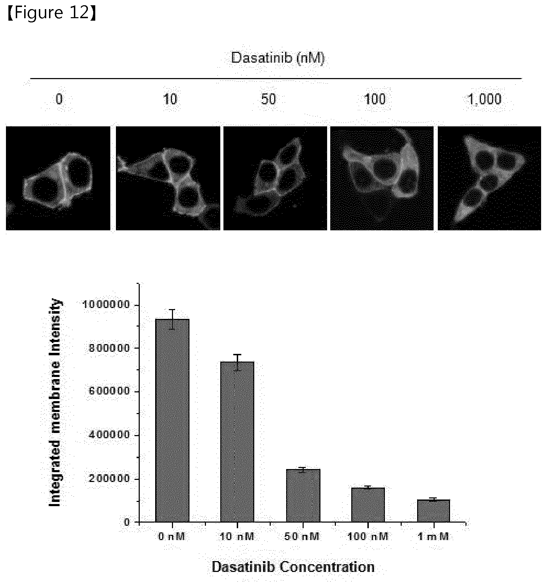

FIG. 12 illustrates the binding inhibitory effect of original unbiotinylated dasatinib on CSK protein which is translocated to the membrane by dasatinib-biotin (Dasatinib: unbiotinylated dasatinib).

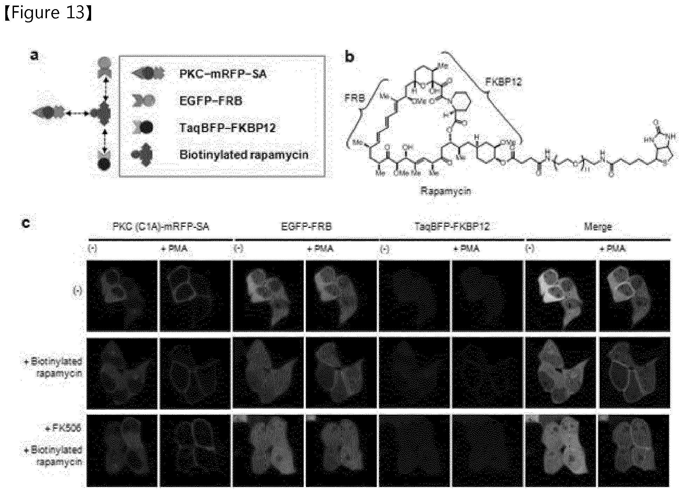

FIG. 13 shows (a) a schematic view of FRB and FKBP proteins of which the binding is detected by rapamycin-biotin, (b) a structure of rapamycin-biotin, and (c) the translocation to the plasma membrane and the translocation inhibitory effect by FK506 ((-) on the horizontal axis: before treatment with signal material (PMA), +PMA: after treatment with signal material, (-) on the vertical axis: rapamycin-biotin untreated group, FK506: tacrolimus, FKBP12: FK506 binding protein 12, FRB: FKBP12-rapamycin binding domain, PCK(C1A)-mRFP-SA: detection images of a first construct comprising streptavidin and RFP, EGFP-FRB: detection images of a third construct comprising GFP and FRB, TagBFP-FKBP12: detection images of a third construct including BFP and FKBP12, Merge: merging of the detection images of PCK(C1A)-mRFP-SA, EGFP-FRB, and TagBFP-FKBP12).

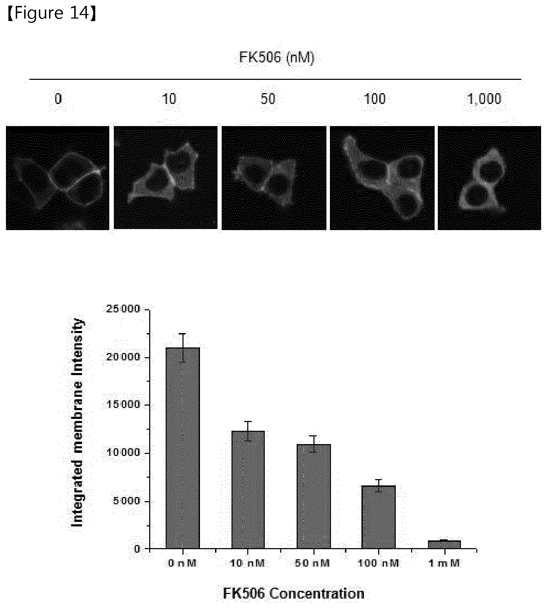

FIG. 14 illustrates results for verifying the inhibition of the translocation of FRB protein depending on the concentration of FK506.

### [Mode for Carrying Out the Invention]

Hereinafter, the present invention will be described in detail by referring to the examples.

However, the following examples are for illustrative purposes only and are not intended to limit the scope of the present invention.

### <Example 1>

### Animal cell lines and transformation thereof

### <1-1> Animal cell line and culturing

CHO-k1 (ATCC CCL-61, *Cricetulus griseus,* hamster, Chinese), HEK293 (ATCC CRL-1573, *Homo sapiens,* human), HeLa (ATCC CCL-2, *Homo sapiens,* human) and SH-SY5Y (ATCC CRL-2266, *Homo sapiens,* human) cell lines were used. The animal cells were cultured according to the instructions of ATCC (American Type Culture Collection) for the individual cells. CHO-k1 cells were cultured by using F-12 medium, while HEK293, HeLa and SH-SY5Y cells were cultured using DMEM medium. Other culturing conditions were the same. Culturing conditions commonly shared for the said cells as follows (Those skilled in the art may modify the specific conditions depending on purposes.). The cells were cultured in pH 7.4 medium (F-12 and DMEM) containing 25 mM HEPES, 10% fetal bovine serum (FBS, v/v), 100 units/ml penicillin and 100 µg/ml streptomycin in a 5% CO₂ incubator maintained at 37 °C.

### <1-2> Transformation of cell lines

In the Examples of the present invention, genes were introduced into the cells using ExGene 500 (Fermentas Life Science), one of the liposome-based techniques. All the conditions for the gene introduction including gene concentration were pursuant to the manufacturer's instructions. More specifically, after transferring the subcultured cells to a 12-well plate with a cover slip, followed by culturing for a day, the culture medium was replaced with 0.9 ml of fresh medium. About 1 µg of the transformation sample was added to 0.1 ml of 150 mM NaCl solution. After completely mixing, 3.3 µl of ExGene reagent was added and mixed by vortexing for 15 seconds. The resultant solution was allowed to stand at room temperature for 10 minutes and then added to each well of the 12-well plate in which the cells were growing. The cells were allowed to be transformed by culturing for 18 hours.

### <Example 2>

### Design and preparation of a first construct, a second construct and a third construct

### <2-1> Design and preparation of first construct

A first construct according to the present invention is a fusion construct composed of a translocation module capable of moving a protein uniformly expressed in the cytoplasm of a cell toward the plasma membrane, a first labeling material analyzable by using a microscope, and a first medium capable of binding to a second medium.

In the present example, protein kinase C was used as a translocation module, mRFP as a first labeling material, and streptavidin as a first medium.

The translocation module was cloned by PCR techniques using the pCMV-SPORT6-PRKCD vector (GenBank accession No. BC043350; purchased from Openbiosystem (http://www.openbiosystems.com/); Catalog No. EHS1001-410108-BC043350) as a template, and then inserted at the NheI/AgeI site of the pmRFP-C3 vector (mRFP; GenBank accession No. DQ903889, SEQ ID NO: 14).

Streptavidin as a first medium was cloned by PCR techniques using GenBank Acc. No. X03591 (SEQ ID NO: 13) as a template, and then inserted at the EcoRI/BamHI site of the pmRFP-C3 vector (mRFP; GenBank accession No. DQ903889, SEQ ID NO: 14).

### <2-2> Design and construction of a second construct

A second construct is composed of a second medium capable of binding to the first medium and a bait.

In the present example, biotin was used as a second medium for streptavidin, while dasatinib and rapamycin were used as a bait. Commercialized dasatinib and rapamycin were respectively substituted with an amine group, and then commercialized NHS-Dpeg12-biotin was allowed to chemically bind thereto, thereby synthesizing dasatinib-biotin and rapamycin-biotin, respectively (*See* FIG. 4).

### <2-3> Design and preparation of third construct

The third construct comprises a prey which has characteristic for binding to the bait of the second construct and a labeling material for analyzing the movement of the prey. The third construct was prepared using a fluorescent material other than used in the first construct. Using green fluorescent protein (EGFP, AzG), red fluorescent protein (mRFP) and infrared fluorescent protein (HcR), the third construct was prepared by the same method described for the first construct.

When EGFP was used as the second labeling material, a pEGFP-C3 vector (Clontech; SEQ ID NO: 15) was used. When mRFP was used, a pmRFP-C3 vector was used. When AzG was used, a pAzG-C3 vector was used. When HcR was used, a pHcR-C3 vector was used. The C3 vectors had been prepared by substituting the EGFP gene sequence site of the pEGFP-C3 vector with AzG and HcR genes, as follows.

The pAzG-C3 vector was prepared as follows. PCR was carried out using a pPM-mAG1 vector (purchased from MBL, Catalog No. AM-V0203; Karasawa, S., et al. 2003, J. Biol. Chem. 278, 34167-34171) as a template and using SEQ ID NO: 16 (AzG-F: 5'-GGCACCGGTCGCCACCATGGACCCCATGGTGAGTGTGAT-3') and SEQ ID NO: 17 (AzG-R: 5'-GGCAGATCTGACAGCTTGGCCTGACTCGGCAGCAT-3') as primers. Then, the EGFP nucleotide sequence of the pEGFP-C3 vector was substituted at the AgeI/NotI site by the resultant PCR product.

The pHcR-C3 vector was prepared as follows. PCR was carried out using pHcRed-Tandem-N1 (purchased from Avrogen, Catalog No. FP204; Gurskaya et al., 2001, FEBS Lett. 507, 16-20.) as a template and using SEQ ID NO: 18 (HcR-F: 5'-GCCACCGGTCGCCACCATGGTGAG-3') and SEQ ID NO: 19 (HcR-R: 5'-GCCGCGGCCGCTTATCAGTTGGCCTTCTCGGGCAGGTC-3') as primers. Then, the EGFP nucleotide sequence of the pEGFP-C3 vector was substituted at the AgeI/NotI site by the resultant PCR product.

### <Example 3>

### Verification of interactions of the first construct, the second construct and the third construct

### <3-1> Verification of expression of constructs and analysis of translocation characteristics

A cover slip containing the cells in which the first construct and the second construct vectors had been introduced was fixed to a perfusion chamber and mounted on the object stage of a confocal laser fluorescence microscope (Carl Zeiss LSM510). Images of the construct vectors were taken before and after external stimulation (treatment with 1 µM PMA).

As for the confocal laser fluorescence microscope, 488 nm argon laser (EGFP or AzG), 543 nm HeNe laser (mRFP) or 561 nm DPSS laser (HcR) was used to induce the excitation of the fluorescent label, and the fluorescence signal generated by each fluorescent label was filtered through the band path filter BP505-530 (EGFP or AzG), long path filter LP560 or BP560-630 (mRFP) or long path filter LP650 (HcR). Images were taken after completely removing the interference between the fluorescences.

An experiment for verifying a basic concept of the present invention that a compound (Oregon green) bound to biotin as the second medium of the second construct can be translocated to the plasma membrane by streptavidin as the first medium attached to the first construct was conducted. As a result, the translocation of the second construct to the plasma membrane by the translocation of the first construct was confirmed through images (*See* FIG. 2).

### <Example 4>

### Real-time analysis of intracellular binding using the first construct, the second construct, and the third construct

For the verification of drug targets of dasatinib used as an anticancer drug, experiments of analyzing the binding of dasatinib with CSK, SRC, EPHA4, and RIPK2 proteins, which have been known as proteins binding to dasatinib, were conducted.

As a result, as can be seen from FIG. 5, the respective proteins (green) were simultaneously translocated to the plasma membrane by PMA as a stimulation for translocation while the first construct (red) and the second construct (dasatinib-biotin) were present.

For the verification of the intracellular binding with respect to CSK, SRC, EPHA4, and RIPK2 proteins, the first construct PKC-mRFP-streptavidin, the third construct GFP-protein expressing vector, and the second construct dasatinib-biotin were prepared as follows. The first construct PKC-mRFP-streptavidin was prepared according to the method of Example <2-1>. The second construct dasatinib-biotin was prepared according to the method of Example <2-2>. The third construct EGFP-protein was prepared by PCR amplification using a human-derived gene as a template, which is obvious to those skilled in the art, and then inserting the resultant PCR product into the pEGFP-C3 vector.

The first construct (PKC-mRFP-streptavidin) and the third construct (EGFP-protein) were introduced into CHO-k1 cells by the methods described in Example <1-2> (ExGene 500), and then allowed to be expressed in an environment into which the second construct (dasatinib-biotin) was introduced. After the 18-hour culturing and 1 uM PMA treatment for 5 minutes, the fluorescent distribution was observed as in Example <3-1>.

The second construct dasatinib-biotin was introduced into a medium in which cells expressing the first construct and the second construct were grown, and then was allowed to infiltrate into the cells by culturing for 4 hours.

As a result, it can be seen that the fluorescence of the third construct (green) to which the prey binding to dasatinib was bound was translocated to the plasma membrane at the time of PMA treatment, like in the red fluorescence (the first construct) to which the translocation module was bound (FIGS. 5, 6, 7, 8, 9, 10, and 12)

On the contrary, as for treatment with unbiotinylated dasatinib in its different concentrations, as can be seen from the results shown in FIG. 12, the translocation of CSK protein (third construct, green) to the plasma membrane was inhibited by dasatinib in a concentration-dependent manner.

The proteins bound to dasatinib as a compound were confirmed through imaging analysis, and the binding of the respective proteins was again verified through immunoprecipitation (IP), which is obvious to those skilled in the art (*See* lower panels of FIGS. 8, 9, and 10, I: Input, B: biotin, D: biotin-dasatinib). As can be seen from FIG. 11, the phosphorylation activity of the biotinylated compound (dasatinib) was very similar to that of the original unbiotinylated compound. Therefore, it was confirmed that a prey protein can be identified under physiological conditions in which the present invention does not affect the change in drug target activity.

### <Example 5>

### Verification of other compound-protein binding for the generalization of the present invention

The above-described examples verified the effectiveness of the present invention. Also, the generalization of the methods of the present invention was confirmed by using other compounds (*See* FIG. 13). When the biotin was bound to rapamycin as another anticancer drug, it was confirmed that FRB and FKBP12 proteins which have been known to bind with rapamycin were simultaneously detected, and the binding was inhibited by another compound FK506 which has been known as a competitive inhibitor against rapamycin. This verifies that the protein binding changes depending on the change in concentration of the competitive inhibitor, and thus the target protein FRB and FKBP12 specifically bind to rapamycin-biotin.

### [Industrial applicability]

As set forth above, the present invention provides a method as defined in the claims for screening a prey which interacts with a bait, the method including: (a) preparing a cell which expresses (i) a first fusion construct comprising a translocation module, a first labeling material, and a first medium, and (ii) a third fusion construct comprising a prey and a second labeling material; (b) introducing (iii) a second fusion construct into the prepared cell, the second construct comprising a bait and a second medium binding with the first medium; (c) allowing the prey and the bait to interact each other; and (d) inducing a translocation of the translocation module via an intrinsic signaling mechanism or by treating with a signaling material that generates an external signal; and (e) confirming the interaction between the prey and the bait by detecting intracellular distributions of the first construct and the third construct. Further, the present invention provides a cell as defined in the claims expressing the first construct and the third construct. Further, the present invention provides a screening kit as defined in the claims comprising the cell and the second construct. The methods of the present invention can overcome disadvantages including inaccuracy and complexity of the existing techniques for biomaterial interaction detection, and can measure the interaction between a material which is not directly produced through gene translation and gene expression in the cell and a material expressed in the cell, and thus are effective in detecting interactions of a broad range of biomaterials so that the present invention is highly industrially applicable.

## Claims

1. A method for screening a prey which interacts with a bait, the method including
(a) preparing a cell which expresses (i) a first fusion construct comprising a translocation module, a first labeling material, and a first medium, and (ii) a third fusion construct comprising a prey and a second labeling material;
(b) introducing (iii) a second fusion construct into the prepared cell, the second construct comprising a bait and a second medium binding with the first medium;
(c) allowing the prey and the bait to interact each other;
(d) inducing a translocation of the translocation module via an intrinsic signaling mechanism or by treating with a signaling material that generates an external signal; and
(e) confirming the interaction between the prey and the bait by detecting intracellular distributions of the first construct and the third construct,
wherein the bait of the second fusion construct and the prey of the third fusion construct are independently selected from the group consisting of protein, polypeptide, polysaccharide, polynucleotide, small organic molecule, synthetic compound, chemical compound and a combination thereof,
wherein the first labeling material and the second labeling material are a material capable of generating a signal that can be distinctively detected, and wherein the first labeling material and the second labeling material are independently selected from the group consisting of a fluorescent material, a ligand, a light-emitting material, a microparticle, a redox material and a radioactive isotope,
wherein the translocation module is selected from the group consisting of protein kinase C (PKC) represented by an amino acid sequence selected from the group consisting of SEQ ID NO: 1, SEQ ID NO: 3, SEQ ID NO: 5, and SEQ ID NO: 7,
wherein the signaling material is selected from the group consisting of phorbol-12-myristate 13-acetate (PMA; phorbol ester), 12-0-tetradecanoylphorbol-13-acetate (TPA), phorbol-12,13-dibutyrate (PDBu), adenosine triphosphate (ATP), tridecanoic acid, arachidonic acid, linoleic acid, DiC8, 130C937, protein kinase C (PKC) activation-related growth factors, and other protein kinase C (PKC) activating materials,
wherein the first medium of the first fusion construct and the second medium of the second fusion construct have high binding specificity and strength therebetween, wherein the first medium is a material capable of being directly produced through gene translation and expression in the cell, and wherein the second medium is a material having a small molecular weight capable of easily penetrating into the cell.

2. The method of claim 1, wherein in step (d), the translocation of the translocation module is induced by treatment with the signaling material.

3. The method of claim 1 or 2, wherein the first labeling material is selected from the group consisting of green fluorescent protein (GFP), enhanced green fluorescent protein (EGFP), red fluorescent protein (RFP), monomeric red fluorescent protein (mRFP), Discosoma sp. red fluorescent protein (DsRed), cyan fluorescent protein (CFP), cyan green fluorescent protein (CGFP), yellow fluorescent protein (YFP), azami green (AzG), Heteractis crispa red fluorescent protein (HcR, HcRed), and blue fluorescent protein (BFP).

4. The method of claim 1 or 2, wherein the second labeling material is selected from the group consisting of green fluorescent protein (GFP), enhanced green fluorescent protein (EGFP), red fluorescent protein (RFP), monomeric red fluorescent protein (mRFP), Discosoma sp. red fluorescent protein (DsRed), cyan fluorescent protein (CFP), cyan green fluorescent protein (CGFP), yellow fluorescent protein (YFP), azami green (AzG), Heteractis crispa red fluorescent protein (HcR, HcRed), and blue fluorescent protein (BFP).

5. The method of claim 1 or 2, wherein the treatment with a signal material is performed by 50 nM to 5 uM of phorbol 12-myristate 13-acetate (PMA, phorbol ester).

6. The method of claim 1 or 2, wherein the first medium is streptavidin and the second medium is biotin.

7. The method of claim 1 or 2, wherein the first medium is dihydrofolate reductase (DHFR) and the second medium is methotrexate (MTX).

8. A cell expressing (i) a first fusion construct comprising a translocation module, a first labeling material, and a first medium; and (ii) a third fusion construct comprising a prey and a second labeling material,
wherein the translocation module is selected from the group consisting of protein kinase C (PKC) represented by an amino acid sequence selected from the group consisting of SEQ ID NO: 1, SEQ ID NO: 3, SEQ ID NO: 5, and SEQ ID NO: 7,
wherein the first labeling material and the second labeling material are a material capable of generating a signal that can be distinctively detected, and wherein the first labeling material and the second labeling material are independently selected from the group consisting of a fluorescent material, a ligand, a light-emitting material, a microparticle, a redox material and a radioactive isotope,
wherein the first medium is a material capable of being directly produced through gene translation and expression in the cell and is selected from the group consisting of streptavidin, dihydrofulate reducatase (DHFR) and a histidine polymer (His-tag), wherein the first medium binds with a second medium of a second fusion construct comprising a bait and a second medium when a second fusion construct is introduced into the cell,
wherein the second medium is a material having a small molecular weight capable of easily penetrating into the cell and is selected from the group consisting of biotin, methotrexate (MTX) and nickle-nitrilitriacetic acid (Ni-NTA),
wherein the bait of the second fusion construct and the prey of the third fusion construct is independently selected from the group consisting of protein, polypeptide, polysaccharide, polynucleotide, small organic molecule, synthetic compound, chemical compound and a combination thereof.

9. The cell of claim 8, wherein the cell is selected from the group consisting of CHO-k1 cell, HEK293 cell, HeLa cell, SH-SY5Y cell, Swiss 3T3 cell, 3T3-L1 cell, NIH/3T3 cell, L-929 cell, Rat2 cell, RBL-2H3 cell, and MDCK cell.

10. The cell of claim 8, wherein the first medium is streptavidin or dihydrofolate reductase (DHFR).

11. A kit for screening a prey interacting with a bait, the kit comprising a cell; and a second fusion construct comprising a second medium binding with a first medium and a bait,
Wherein the cell expresses (i) a first fusion construct comprising a translocation module, a first labeling material, and a first medium; and (ii) a third fusion construct comprising a prey and a second labeling material,
wherein the translocation module is selected from the group consisting of protein kinase C (PKC) represented by an amino acid sequence selected from the group consisting of SEQ ID NO: 1, SEQ ID NO: 3, SEQ ID NO: 5, and SEQ ID NO: 7,
wherein the first labeling material and the second labeling material are a material capable of generating a signal that can be distinctively detected, and wherein the first labeling material and the second labeling material are independently selected from the group consisting of a fluorescent material, a ligand, a light-emitting material, a microparticle, a redox material and a radioactive isotope,
wherein the first medium is a material capable of being directly produced through gene translation and expression in the cell and is selected from the group consisting of streptavidin, dihydrofulate reducatase (DHFR) and a histidine polymer (His-tag), wherein the first medium binds with the second medium of the second fusion construct comprising the bait and the second medium when the second fusion construct is introduced into the cell,
wherein the second medium is a material having a small molecular weight capable of easily penetrating into the cell and is selected from the group consisting of biotin, methotrexate (MTX) and nickle-nitrilitriacetic acid (Ni-NTA), wherein the bait of the second fusion construct and the prey of the third fusion construct is independently selected from the group consisting of protein, polypeptide, polysaccharide, polynucleotide, small organic molecule, synthetic compound, chemical compound and a combination thereof.

12. The kit of claim 11, wherein the first medium is streptavidin and the second medium is biotin.

13. The kit of claim 11, wherein the first medium is dihydrofolate reductase (DHFR) and the second medium is methotrexate (MTX).

14. The kit of claim 11, further comprising a signalling material for allowing the prey and the bait to interact each other,
wherein the signaling material is selected from the group consisting of phorbol-12-myristate 13-acetate (PMA; phorbol ester), 12-O-tetradecanoylphorbol-13-acetate (TPA), phorbol-12,13-dibutyrate (PDBu), adenosine triphosphate (ATP), tridecanoic acid, arachidonic acid, linoleic acid, DiC8, 130C937, protein kinase C (PKC) activation-related growth factors, and other protein kinase C (PKC) activating materials,.

## Patentansprüche

1. Verfahren zum Überprüfen einer Beute, welche mit einem Köder interagiert, umfassend:
(a) Herstellen einer Zelle, welche (i) ein erstes Fusionskonstrukt umfassend ein Translokationsmodul, ein erstes Bezeichnungsmaterial und ein erstes Medium, und (ii) ein drittes Fusionskonstrukt umfassend eine Beute und ein zweites Bezeichnungsmaterial exprimiert;
(b) Einführen (iii) eines zweiten Fusionskonstruktes in die hergestellte Zelle, wobei das zweite Konstrukt einen Köder und ein zweites Medium, welches sich mit dem ersten Medium bindet, umfasst;
(c) Gewähren eines Interagierens zwischen der Beute und dem Köder;
(d) Induzieren einer Translokation über das Translokationsmodul über einen intrinsischen Signalisierungsmechanismus oder durch Behandeln mit einem Signalisierungsmaterial, welches ein externes Signal erzeugt; und
(e) Bestätigen der Interaktion zwischen der Beute und dem Köder durch Detektieren intrazellulärer Verteilungen des ersten Konstruktes und des zweiten Konstruktes,
wobei der Köder des zweiten Fusionskonstruktes und die Beute des dritten Fusionskonstruktes unabhängig ausgewählt sind aus der Gruppe bestehend aus einem Protein, einem Polypeptid, einem Polysaccharid, einem Polynucleotid, einem kleinen organischen Molekül, einer synthetischen Verbindung, einer chemischen Verbindung und einer Kombination davon,
wobei das erste Bezeichnungsmaterial und das zweite Bezeichnungsmaterial fähig sind, ein Signal, welches unterschiedlich detektiert werden kann, zu erzeugen, und wobei das erste Bezeichnungsmaterial und das zweite Bezeichnungsmaterial unabhängig ausgewählt sind aus der Gruppe bestehend aus einem fluoreszenten Material, einem Liganden, einem licht-emittierenden Material, einem Mikropartikel, einem Redoxmaterial und einem radioaktiven Isotop, wobei das Translokationsmodul ausgewählt ist aus der Gruppe bestehend aus einer Proteinkinase C (PKC), welche durch eine Aminosäurensequenz ausgewählt aus der Gruppe bestehend aus SEQ ID NO: 1, SEQ ID NO: 3, SEQ ID NO:5 und SEG ID NO: 7 dargestellt wird,
wobei das Signalisierungsmaterial ausgewählt ist aus der Gruppe bestehend aus Phorbol-12-myristat-13-acetat (PMA; Phorbolester), 12-O-Tetradecanoylphorbol-13-acetat (TPA), Phorbol-12,13-dibutyrat (PDBu), Adenosintriphosphat (ATP), Tridecansäure, Arachidonsäure, Linolsäure, DiC8, 130C937, Proteinkinase C (PKC), Aktivierung entgegennehmenden Wachstumsfaktoren und anderen Proteinkinase C (PKC)-aktivierenden Materialien,
wobei das erste Medium des ersten Fusionskonstruktes und das zweite Medium des zweiten Fusionskonstruktes eine hohe Bindungsspezifität- und Stärke dazwischen aufweisen, wobei das erste Medium ein Material ist, welches fähig ist, direkt durch Gentranslation- und Expression in der Zelle hergestellt zu werden, und wobei das zweite Medium ein Material mit einem geringen Molekulargewicht ist, welches fähig ist, einfach in die Zelle einzudringen.

2. Verfahren nach Anspruch 1, wobei in Schritt (d) die Translokation des Translokationsmoduls durch eine Behandlung mit dem Signalisierungsmaterial induziert wird.

3. Verfahren nach Anspruch 1 oder 2, wobei das erste Bezeichnungsmaterial ausgewählt ist aus der Gruppe bestehend aus einem grün fluoreszierenden Protein (GFP), einem erweiterten grün fluoreszierenden Protein (EGFP), einem rot fluoreszierenden Protein (RFP), einem monomeren rot fluoreszierenden Protein (mRFP), einem Discosoma sp. roten fluoreszierenden Protein (DsRed), einem zyan fluoreszierenden Protein, einem gelb fluoreszierenden Protein (YFP), einem Azamigrün (AzG), einem Heteractis crispa roten fluoreszierenden Protein (HcR, HcRed) und einem blauen fluoreszierenden Protein (BFP).

4. Verfahren nach Anspruch 1 oder 2, wobei das zweite Bezeichnungsmaterial ausgewählt ist aus der Gruppe bestehend aus einem grünen fluoreszierenden Protein (GFP), einem erweiterten grün fluoreszierenden Protein (EGFP), einem rot fluoreszierenden Protein (RFP), einem monomeren rot fluoreszierenden Protein (mRFP), einem Discosoma sp. roten fluoreszierenden Protein (DsRed), einem zyan fluoreszierenden Protein, einem gelb fluoreszierenden Protein (YFP), einem Azamigrün (AzG), einem Heteractis crispa roten fluoreszierenden Protein (HcR, HcRed) und einem blauen fluoreszierenden Protein (BFP).

5. Verfahren nach Anspruch 1 oder 2, wobei die Behandlung mit einem Signalisierungsmaterial mit 50 nM bis 5 uM Phorbol-12-myristat-13-acetat (PMA, Phorbolester) durchgeführt wird.

6. Verfahren nach Anspruch 1 oder 2, wobei das erste Medium Streptavidin und das zweite Medium Biotin ist.

7. Verfahren nach Anspruch 1 oder 2, wobei das erste Medium Dihydrofolatreduktase (DHFR) und das zweite Medium Methotrexat (MTX) ist.

8. Zelle, welche (i) ein erstes Fusionskonstrukt umfassend ein Translokationsmodul, ein erstes Bezeichnungsmaterial und ein erstes Medium; und (ii) ein drittes Fusionskonstrukt, welches eine Beute und ein zweites Bezeichnungsmaterial aufweist, exprimiert,
wobei das Translokationsmodul ausgewählt ist aus der Gruppe bestehend aus einer Proteinkinase (PKC), welche durch eine Aminosäuresequenz ausgewählt aus der Gruppe bestehend aus SEQ ID NO: 1, SEQ ID NO: 3, SEQ ID NO:5 und SEG ID NO: 7 dargestellt wird,
wobei das erste Bezeichnungsmaterial und das zweite Bezeichnungsmaterial ein Material sind, das fähig ist, ein Signal, welches unterschiedlich detektiert werden kann, zu erzeugen und wobei das erste Bezeichnungsmaterial und das zweite Bezeichnungsmaterial unabhängig ausgewählt ist aus der Gruppe bestehend aus einem fluoreszenten Material, einem Liganden, einem licht-emittierenden Material, einem Mikropartikel, einem Redoxmaterial und einem radioaktiven Isotop, wobei das erste Medium ein Material ist, welches fähig ist,, direkt durch Gentranslation- und Expression in der Zelle produziert zu werden und ausgewählt ist aus der Gruppe bestehend aus Strepavidin, Dihydrofolatreduktase (DHFR) und einem Histidinpolymer (His-tag), wobei das erste Medium sich mit dem zweiten Medium eines zweiten Fusionskonstruktes umfassend ein Köder und einem weiten Medium ,wenn ein zweites Fusionskonstrukt in die Zelle eingeführt wird, bindet, wobei das zweite Medium ein Material ist, welches ein geringes Molekulargewicht aufweist und fähig ist, einfach in die Zelle einzudringen und ausgewählt ist aus der Gruppe bestehend aus Biotin, Methotrexat (MTX) und Nickelnitriltriessigsäure (Ni-NTA),
wobei der Köder des zweiten Fusionskonstruktes und die Beute des dritten Fusionskonstruktes unabhängig ausgewählt ist aus der Gruppe bestehend aus einem Protein, einem Polypeptid, einem Polysaccharid, einem Polynucleotid, einem kleinen organischen Molekül, einer synthetischen Verbindung, einer chemischen Verbindung und einer Kombination davon.

9. Zelle nach Anspruch 8, wobei die Zelle ausgewählt ist aus der Gruppe bestehend aus einer CHO-k1-Zelle, einer HEK293-Zelle, einer HeLa-Zelle, einer SH-SY5Y-Zelle, einer Swiss 3T3-Zelle, einer 3T3-L1-Zelle, einer NIH/3T3-Zelle, einer L-929-Zelle, einer Rat2-Zelle, einer RBL-2H3-Zelle und einer MDCK-Zelle.

10. Zelle nach Anspruch 8, wobei das erste Medium Streptavidin oder Dihydrofolatreduktase (DHFR) ist.

11. Set zum Überprüfen einer Beute, welche mit einem Köder interagiert, wobei das Set eine Zelle; und ein zweites Fusionskonstrukt umfassend ein zweites Medium, welches sich mit dem ersten Medium und einem Köder bindet,
wobei die Zelle (i) ein erstes Fusionskonstrukt umfassend ein Translokationsmodul, ein erstes Bezeichnungsmaterial und ein erstes Medium; und (ii) ein drittes Fusionskonstrukt umfassend eine Beute und ein zweites Bezeichnungsmaterial exprimiert,
wobei das Translokationsmodul ausgewählt ist aus der Gruppe bestehend aus Proteinkinase C (PKC) welche durch eine Aminosäuresequenz ausgewählt aus der Gruppe bestehend aus SEQ ID NO: 1, SEQ ID NO: 3, SEQ ID NO:5 und SEG ID NO: 7 dargestellt wird,
wobei das erste Bezeichnungsmaterial und das zweite Bezeichnungsmaterial ein Material sind, welches fähig ist, ein Signal, welches unterschiedlich detektiert werden kann, zu erzeugen, und wobei das erste Bezeichnungsmaterial und das zweite Bezeichnungsmaterial unabhängig ausgewählt sind aus der Gruppe bestehend aus einem fluoreszenten Material, einem Liganden, einem lichtemittierenden Material, einem Mikropartikel, einem Redoxmaterial und einem radioaktiven Isotop,
wobei das erste Medium ein Material ist, welches fähig ist, direkt durch Gentranslation- und Expression in der Zelle hergestellt zu werden und ausgewählt ist aus der Gruppe bestehend aus Streptavidin, Dihydrofolatreduktase (DHFR) und Histidinpolymer (His-tag), wobei das erste Medium sich mit dem zweiten Medium des zweiten Fusionskonstruktes umfassend den Köder und das zweite Medium, wenn das zweite Fusionskonstrukt in die Zelle eingeführt wird, bindet,
wobei das zweite Medium ein Material ist, welches ein geringes Molekulargewicht aufweist und fähig ist, einfach in die Zelle einzudringen und ausgewählt ist aus der Gruppe bestehend aus Biotin, Methotrexat (MTX) und Nickelnitriltriessigsäure (Ni-NTA),
wobei der Köder und das zweite Fusionskonstrukt und die Beute des dritten Fusionskonstruktes unabhängig ausgewählt sind aus der Gruppe bestehend aus einem Protein, einem Polypeptid, einem Polysaccharid, einem Polynucleotid, einem kleinen organischen Molekül, einer synthetischen Verbindung, einer chemischen Verbindung oder einer Kombination davon.

12. Set nach Anspruch 11, wobei das erste Medium Streptavidin ist und das zweite Medium Biotin ist.

13. Seht nach Anspruch 11, wobei das erste Medium Dihydrofolatreduktase (DHFR) und das zweite Medium Methotrexat (MTX) ist.

14. Set nach Anspruch 11, ferner umfassend ein Signalisierungsmaterial um der Beute und dem Köder zu erlauben miteinander zu interagieren, wobei das Signalisierungsmaterial ausgewählt ist aus der Gruppe bestehend aus Phorbol-12-myristat-13-acetat (PMA; Phorbolester), 12-O-Tetradecanoylphorbol-13-acetat (TPA), Phorbol-12,13-dibutyrat (PDBu), Adenosintriphosphat (ATP), Tridecansäure, Arachidonsäure, Linolsäure, DiC8, 130C937, Proteinkinase C (PKC), Aktivierung entgegennehmenden Wachstumsfaktoren und anderen Proteinkinase C (PKC)-aktivierenden Materialien.

## Revendications

1. Procédé de criblage d'une proie qui interagit avec un appât, le procédé comportant le fait
(a) de préparer une cellule qui exprime (i) une première construction de fusion comprenant un module de translocation, un premier matériau de marquage et un premier milieu, et (ii) une troisième construction de fusion comprenant une proie et un deuxième matériau de marquage ;
(b) d'introduire (iii) une deuxième construction de fusion dans la cellule préparée, la deuxième construction comprenant un appât et un deuxième milieu se liant au premier milieu ;
(c) de permettre à la proie et à l'appât d'interagir entre eux ;
(d) d'induire une translocation du module de translocation par un mécanisme de signalisation intrinsèque ou par traitement avec un matériau de signalisation qui génère un signal externe ; et
(e) de confirmer l'interaction entre la proie et l'appât en détectant des distributions intracellulaires de la première construction et de la troisième construction,
dans lequel l'appât de la deuxième construction de fusion et la proie de la troisième construction de fusion sont indépendamment choisis dans le groupe constitué d'une protéine, d'un polypeptide, d'un polysaccharide, d'un polynucléotide, d'une petite molécule organique, d'un composé synthétique, d'un composé chimique et d'une combinaison de ceux-ci,
dans lequel le premier matériau de marquage et le deuxième matériau de marquage sont un matériau capable de générer un signal qui peut être distinctement détecté, et dans lequel le premier matériau de marquage et le deuxième matériau de marquage sont indépendamment choisis dans le groupe constitué d'un matériau fluorescent, d'un ligand, d'un matériau électroluminescent, d'une microparticule, d'un matériau rédox et d'un isotope radioactif,
dans lequel le module de translocation est choisi dans le groupe constitué de la protéine kinase C (PKC) représentée par une séquence d'acides aminés choisie dans le groupe constitué des séquences SEQ ID NO : 1, SEQ ID NO : 3, SEQ ID NO : 5 et SEQ ID NO : 7,
dans lequel le matériau de signalisation est choisi dans le groupe constitué du phorbol-12-myristate 13-acétate (PMA; ester de phorbol), du 12-O-tétradécanoylphorbol-13-acétate (TPA), du phorbol-12,13-dibutyrate (PDBu), de l'adénosine triphosphate (ATP), de l'acide tridécanoïque, de l'acide arachidonique, de l'acide linoléique, DiC8, 130C937, des facteurs de croissance liés à l'activation de la protéine kinase C (PKC) et d'autres matériaux d'activation de la protéine kinase C (PKC),
dans lequel le premier milieu de la première construction de fusion et le deuxième milieu de la deuxième construction de fusion ont une spécificité et une force de liaison élevées entre eux, dans lequel le premier milieu est un matériau capable d'être directement produit par la traduction et l'expression de gène dans la cellule, et dans lequel le deuxième milieu est un matériau ayant un faible poids moléculaire capable de pénétrer facilement dans la cellule.

2. Procédé de la revendication 1, dans lequel, dans l'étape (d), la translocation du module de translocation est induite par traitement avec le matériau de signalisation.

3. Procédé de la revendication 1 ou 2, dans lequel le premier matériau de marquage est choisi dans le groupe constitué de protéine fluorescente verte (GFP), de protéine fluorescente verte améliorée (EGFP), de protéine fluorescente rouge (RFP), de protéine fluorescente rouge monomère (mRFP), de protéine fluorescente rouge de Discosoma sp. (DsRed), de protéine fluorescente cyan (CFP), de protéine fluorescente vert-cyan (CGFP), de protéine fluorescente jaune (YFP), de vert-azami (AzG), de protéine fluorescente rouge d'Heteractis crispa (HcR, HcRed) et de protéine fluorescente bleue (BFP).

4. Procédé de la revendication 1 ou 2, dans lequel le deuxième matériau de marquage est choisi dans le groupe constitué de protéine fluorescente verte (GFP), de protéine fluorescente verte améliorée (EGFP), de protéine fluorescente rouge (RFP), de protéine fluorescente rouge monomère (mRFP), de protéine fluorescente rouge de Discosoma sp. (DsRed), de protéine fluorescente cyan (CFP), de protéine fluorescente vert-cyan (CGFP), de protéine fluorescente jaune (YFP), de vert-azami (AzG), de protéine fluorescente rouge d'Heteractis crispa (HcR, HcRed) et de protéine fluorescente bleue (BFP).

5. Procédé de la revendication 1 ou 2, dans lequel le traitement avec un matériau de signalisation est effectué par 50 nM à 5 µM de phorbol 12-myristate 13-acétate (PMA, ester de phorbol).

6. Procédé de la revendication 1 ou 2, dans lequel le premier milieu est la streptavidine et le deuxième milieu est la biotine.

7. Procédé de la revendication 1 ou 2, dans lequel le premier milieu est la dihydrofolate réductase (DHFR) et le deuxième milieu est le méthotrexate (MTX).

8. Cellule exprimant (i) une première construction de fusion comprenant un module de translocation, un premier matériau de marquage et un premier milieu ; et (ii) une troisième construction de fusion comprenant une proie et un deuxième matériau de marquage,
dans laquelle le module de translocation est choisi dans le groupe constitué de la protéine kinase C (PKC) représentée par une séquence d'acides aminés choisie dans le groupe constitué des séquences SEQ ID NO : 1, SEQ ID NO : 3, SEQ ID NO : 5 et SEQ ID NO : 7,
dans laquelle le premier matériau de marquage et le deuxième matériau de marquage sont un matériau capable de générer un signal qui peut être distinctement détecté, et dans laquelle le premier matériau de marquage et le deuxième matériau de marquage sont indépendamment choisis dans le groupe constitué d'un matériau fluorescent, d'un ligand, d'un matériau électroluminescent, d'une microparticule, d'un matériau rédox et d'un isotope radioactif,
dans laquelle le premier milieu est un matériau capable d'être directement produit par la traduction et l'expression de gène dans la cellule et est choisi dans le groupe constitué de la streptavidine, de la dihydrofolate réductase (DHFR) et d'un polymère d'histidine (His-tag), dans laquelle le premier milieu se lie à un deuxième milieu d'une deuxième construction de fusion comprenant un appât et un deuxième milieu lorsqu'une deuxième construction de fusion est introduite dans la cellule,
dans laquelle le deuxième milieu est un matériau ayant un faible poids moléculaire capable de pénétrer facilement dans la cellule et est choisi dans le groupe constitué de la biotine, du méthotrexate (MTX) et de l'acide nickel-nitrilotriacétique (Ni-NTA),
dans laquelle l'appât de la deuxième construction de fusion et la proie de la troisième construction de fusion sont indépendamment choisis dans le groupe constitué d'une protéine, d'un polypeptide, d'un polysaccharide, d'un polynucléotide, d'une petite molécule organique, d'un composé synthétique, d'un composé chimique et d'une combinaison de ceux-ci.

9. Cellule de la revendication 8, dans laquelle la cellule est choisie dans le groupe constitué de la cellule CHO-k1, de la cellule HEK293, de la cellule HeLa, de la cellule SH-SY5Y, de la cellule Swiss 3T3, de la cellule 3T3-L1, de la cellule NIH/3T3, de la cellule L-929, de la cellule Rat2, de la cellule RBL-2H3 et de la cellule MDCK.

10. Cellule de la revendication 8, dans laquelle le premier milieu est la streptavidine ou la dihydrofolate réductase (DHFR).

11. Kit de criblage d'une proie interagissant avec un appât, le kit comprenant une cellule ; et une deuxième construction de fusion comprenant un deuxième milieu se liant à un premier milieu et un appât,
dans lequel la cellule exprime (i) une première construction de fusion comprenant un module de translocation, un premier matériau de marquage et un premier milieu ; et (ii) une troisième construction de fusion comprenant une proie et un deuxième matériau de marquage,
dans lequel le module de translocation est choisi dans le groupe constitué de la protéine kinase C (PKC) représentée par une séquence d'acides aminés choisie dans le groupe constitué des séquences SEQ ID NO : 1, SEQ ID NO : 3, SEQ ID NO : 5 et SEQ ID NO : 7,
dans lequel le premier matériau de marquage et le deuxième matériau de marquage sont un matériau capable de générer un signal qui peut être distinctement détecté, et dans lequel le premier matériau de marquage et le deuxième matériau de marquage sont indépendamment choisis dans le groupe constitué d'un matériau fluorescent, d'un ligand, d'un matériau électroluminescent, d'une microparticule, d'un matériau rédox et d'un isotope radioactif,
dans lequel le premier milieu est un matériau capable d'être directement produit par la traduction et l'expression de gène dans la cellule et est choisi dans le groupe constitué de la streptavidine, de la dihydrofolate réductase (DHFR) et d'un polymère d'histidine (His-tag), dans lequel le premier milieu se lie au deuxième milieu de la deuxième construction de fusion comprenant l'appât et le deuxième milieu lorsque la deuxième construction de fusion est introduite dans la cellule,
dans lequel le deuxième milieu est un matériau ayant un faible poids moléculaire capable de pénétrer facilement dans la cellule et est choisi dans le groupe constitué de la biotine, du méthotrexate (MTX) et de l'acide nickel-nitrilotriacétique (Ni-NTA), dans lequel l'appât de la deuxième construction de fusion et la proie de la troisième construction de fusion sont indépendamment choisis dans le groupe constitué d'une protéine, d'un polypeptide, d'un polysaccharide, d'un polynucléotide, d'une petite molécule organique, d'un composé synthétique, d'un composé chimique et d'une combinaison de ceux-ci.

12. Kit de la revendication 11, dans lequel le premier milieu est la streptavidine et le deuxième milieu est la biotine.

13. Kit de la revendication 11, dans lequel le premier milieu est la dihydrofolate réductase (DHFR) et le deuxième milieu est le méthotrexate (MTX).

14. Kit de la revendication 11, comprenant en outre un matériau de signalisation pour permettre à la proie et à l'appât d'interagir entre eux,
dans lequel le matériau de signalisation est choisi dans le groupe constitué du phorbol-12-myristate 13-acétate (PMA; ester de phorbol), du 12-O-tétradécanoylphorbol-13-acétate (TPA), du phorbol-12,13-dibutyrate (PDBu), de l'adénosine triphosphate (ATP), de l'acide tridécanoïque, de l'acide arachidonique, de l'acide linoléique, DiC8, 130C937, des facteurs de croissance liés à l'activation de la protéine kinase C (PKC) et d'autres matériaux d'activation de la protéine kinase C (PKC).


### Sequence Listing

<110> Korea basic science institute
<120> Method for detecting interactions between molecular compound and its binding proteins
<130> OP12-0028
<160> 19
<170> KopatentIn 2.0
<210> 1
   <211> 676
   <212> PRT
   <213> Homo sapiens
<400> 1
   Met Ala Pro Phe Leu Arg Ile Ala Phe Asn Ser Tyr Glu Leu Gly Ser
    1               5                  10                  15
   Leu Gln Ala Glu Asp Glu Ala Asn Gln Pro Phe Cys Ala Val Lys Met
               20                  25                  30
   Lys Glu Ala Leu Ser Thr Glu Arg Gly Lys Thr Leu Val Gln Lys Lys
           35                  40                  45
   Pro Thr Met Tyr Pro Glu Trp Lys Ser Thr Phe Asp Ala His Ile Tyr
       50                  55                  60
   Glu Gly Arg Val Ile Gln Ile Val Leu Met Arg Ala Ala Glu Glu Pro
   65                  70                  75                  80
   Val Ser Glu Val Thr Val Gly Val Ser Val Leu Ala Glu Arg Cys Lys
                   85                  90                  95
   Lys Asn Asn Gly Lys Ala Glu Phe Trp Leu Asp Leu Gln Pro Gln Ala
               100                 105                 110
   Lys Val Leu Met Ser Val Gln Tyr Phe Leu Glu Asp Val Asp Cys Lys
           115                 120                 125
   Gln Ser Met Arg Ser Glu Asp Glu Ala Lys Phe Pro Thr Met Asn Arg
       130                 135                 140
   Arg Gly Ala Ile Lys Gln Ala Lys Ile His Tyr Ile Lys Asn His Glu
   145                 150                 155                 160
   Phe Ile Ala Thr Phe Phe Gly Gln Pro Thr Phe Cys Ser Val Cys Lys
                   165                 170                 175
   Asp Phe Val Trp Gly Leu Asn Lys Gln Gly Tyr Lys Cys Arg Gln Cys
               180                 185                 190
   Asn Ala Ala Ile His Lys Lys Cys Ile Asp Lys Ile Ile Gly Arg Cys
           195                 200                 205
   Thr Gly Thr Ala Ala Asn Ser Arg Asp Thr Ile Phe Gln Lys Glu Arg
       210                 215                 220
   Phe Asn Ile Asp Met Pro His Arg Phe Lys Val His Asn Tyr Met Ser
   225                 230                 235                 240
   Pro Thr Phe Cys Asp His Cys Gly Ser Leu Leu Trp Gly Leu Val Lys
                   245                 250                 255
   Gln Gly Leu Lys Cys Glu Asp Cys Gly Met Asn Val His His Lys Cys
               260                 265                 270
   Arg Glu Lys Val Ala Asn Leu Cys Gly Ile Asn Gln Lys Leu Leu Ala
           275                 280                 285
   Glu Ala Leu Asn Gln Val Thr Gln Arg Ala Ser Arg Arg Ser Asp Ser
       290                 295                 300
   Ala Ser Ser Glu Pro Val Gly Ile Tyr Gln Gly Phe Glu Lys Lys Thr
   305                 310                 315                 320
   Gly Val Ala Gly Glu Asp Met Gln Asp Asn Ser Gly Thr Tyr Gly Lys
                   325                 330                 335
   Ile Trp Glu Gly Ser Ser Lys Cys Asn Ile Asn Asn Phe Ile Phe His
               340                 345                 350
   Lys Val Leu Gly Lys Gly Ser Phe Gly Lys Val Leu Leu Gly Glu Leu
           355                 360                 365
   Lys Gly Arg Gly Glu Tyr Phe Ala Ile Lys Ala Leu Lys Lys Asp Val
       370                 375                 380
   Val Leu Ile Asp Asp Asp Val Glu Cys Thr Met Val Glu Lys Arg Val
   385                 390                 395                 400
   Leu Thr Leu Ala Ala Glu Asn Pro Phe Leu Thr His Leu Ile Cys Thr
                   405                 410                 415
   Phe Gln Thr Lys Asp His Leu Phe Phe Val Met Glu Phe Leu Asn Gly
               420                 425                 430
   Gly Asp Leu Met Tyr His Ile Gln Asp Lys Gly Arg Phe Glu Leu Tyr
           435                 440                 445
   Arg Ala Thr Phe Tyr Ala Ala Glu Ile Met Cys Gly Leu Gln Phe Leu
       450                 455                 460
   His Ser Lys Gly Ile Ile Tyr Arg Asp Leu Lys Leu Asp Asn Val Leu
   465                 470                 475                 480
   Leu Asp Arg Asp Gly His Ile Lys Ile Ala Asp Phe Gly Met Cys Lys
                   485                 490                 495
   Glu Asn Ile Phe Gly Glu Ser Arg Ala Ser Thr Phe Cys Gly Thr Pro
               500                 505                 510
   Asp Tyr Ile Ala Pro Glu Ile Leu Gln Gly Leu Lys Tyr Thr Phe Ser
           515                 520                 525
   Val Asp Trp Trp Ser Phe Gly Val Leu Leu Tyr Glu Met Leu Ile Gly
       530                 535                 540
   Gln Ser Pro Phe His Gly Asp Asp Glu Asp Glu Leu Phe Glu Ser Ile
   545 550 555 560
   Arg Val Asp Thr Pro His Tyr Pro Arg Trp Ile Thr Lys Glu Ser Lys
   565 570 575
   Asp Ile Leu Glu Lys Leu Phe Glu Arg Glu Pro Thr Lys Arg Leu Gly
   580 585 590
   Val Thr Gly Asn Ile Lys Ile His Pro Phe Phe Lys Thr Ile Asn Trp
   595 600 605
   Thr Leu Leu Glu Lys Arg Arg Leu Glu Pro Pro Phe Arg Pro Lys Val
   610 615 620
   Lys Ser Pro Arg Asp Tyr Ser Asn Phe Asp Gln Glu Phe Leu Asn Glu
   625 630 635 640
   Lys Ala Arg Leu Ser Tyr Ser Asp Lys Asn Leu Ile Asp Ser Met Asp
   645 650 655
   Gln Ser Ala Phe Ala Gly Phe Ser Phe Val Asn Pro Lys Phe Glu His
   660 665 670
   Leu Leu Glu Asp
   675
<210> 2
   <211> 2028
   <212> DNA
   <213> Homo sapiens
<400> 2
   atggcgccgt tcctgcgcat cgccttcaac tcctatgagc tgggctccct gcaggccgag 60
   gacgaggcga accagccctt ctgtgccgtg aagatgaagg aggcgctcag cacagagcgt 120
   gggaaaacac tggtgcagaa gaagccgacc atgtatcctg agtggaagtc gacgttcgac 180
   gcccacatct atgaggggcg cgtcatccag attgtgctaa tgcgggcagc agaggagcca 240
   gtgtctgagg tgaccgtggg tgtgtcggtg ctggccgagc gctgcaagaa gaacaatggc     300
   aaggctgagt tctggctgga cctgcagcct caggccaagg tgttgatgtc tgttcagtat     360
   ttcctggagg acgtggattg caaacagtct atgcgcagtg aggacgaggc caagttccca     420
   acgatgaacc gccgcggagc catcaaacag gccaaaatcc actacatcaa gaaccatgag     480
   tttatcgcca ccttctttgg gcaacccacc ttctgttctg tgtgcaaaga ctttgtctgg     540
   ggcctcaaca agcaaggcta caaatgcagg caatgtaacg ctgccatcca caagaaatgc     600
   atcgacaaga tcatcggcag atgcactggc accgcggcca acagccggga cactatattc     660
   cagaaagaac gcttcaacat cgacatgccg caccgcttca aggttcacaa ctacatgagc     720
   cccaccttct gtgaccactg cggcagcctg ctctggggac tggtgaagca gggattaaag     780
   tgtgaagact gcggcatgaa tgtgcaccat aaatgccggg agaaggtggc caacctctgc     840
   ggcatcaacc agaagctttt ggctgaggcc ttgaaccaag tcacccagag agcctcccgg     900
   agatcagact cagcctcctc agagcctgtt gggatatatc agggtttcga gaagaagacc     960
   ggagttgctg gggaggacat gcaagacaac agtgggacct acggcaagat ctgggagggc    1020
   agcagcaagt gcaacatcaa caacttcatc ttccacaagg tcctgggcaa aggcagcttc    1080
   gggaaggtgc tgcttggaga gctgaagggc agaggagagt actttgccat caaggccctc    1140
   aagaaggatg tggtcctgat cgacgacgac gtggagtgca ccatggttga gaagcgggtg    1200
   ctgacacttg ccgcagagaa tccctttctc acccacctca tctgcacctt ccagaccaag    1260
   gaccacctgt tctttgtgat ggagttcctc aacggggggg acctgatgta ccacatccag     1320
   gacaaaggcc gctttgaact ctaccgtgcc acgttttatg ccgctgagat aatgtgtgga    1380
   ctgcagtttc tacacagcaa gggcatcatt tacagggacc tcaaactgga caatgtgctg    1440
   ttggaccggg atggccacat caagattgcc gactttggga tgtgcaaaga gaacatattc      1500
   ggggagagcc gggccagcac cttctgcggc acccctgact atatcgcccc tgagatccta      1560
   cagggcctga agtacacatt ctctgtggac tggtggtctt tcggggtcct tctgtacgag      1620
   atgctcattg gccagtcccc cttccatggt gatgatgagg atgaactctt cgagtccatc      1680
   cgtgtggaca cgccacatta tccccgctgg atcaccaagg agtccaagga catcctggag      1740
   aagctctttg aaagggaacc aaccaagagg ctgggagtga cgggaaacat caaaatccac      1800
   cccttcttca agaccataaa ctggactctg ctggaaaagc ggaggttgga gccacccttc      1860
   aggcccaaag tgaagtcacc cagagactac agtaactttg accaggagtt cctgaacgag      1920
   aaggcgcgcc tctcctacag cgacaagaac ctcatcgact ccatggacca gtctgcattc      1980
   gctggcttct cctttgtgaa ccccaaattc gagcacctcc tggaagat                   2028
<210> 3
   <211> 71
   <212> PRT
   <213> Artificial Sequence
<220>
   <223> TMA
<400> 3
   Met Lys Gln Ala Lys Ile His Tyr Ile Lys Asn His Glu Phe Ile Ala
   1               5                   10                  15
   Thr Phe Phe Gly Gln Pro Thr Phe Cys Ser Val Cys Lys Asp Phe Val
               20                  25                  30
   Trp Gly Leu Asn Lys Gln Gly Tyr Lys Cys Arg Gln Cys Asn Ala Ala
           35                  40                  45
   Ile His Lys Lys Cys Ile Asp Lys Ile Ile Gly Arg Cys Thr Gly Thr
       50                  55                  60
   Ala Ala Asn Ser Arg Asp Thr
   65                  70
<210> 4
   <211> 213
   <212> DNA
   <213> Artificial Sequence
<220>
   <223> TMA
<400> 4
   atgaaacagg ccaaaatcca ctacatcaag aaccatgagt ttatcgccac cttctttggg      60
   caacccacct tctgttctgt gtgcaaagac tttgtctggg gcctcaacaa gcaaggctac     120
   aaatgcaggc aatgtaacgc tgccatccac aagaaatgca tcgacaagat catcggcaga     180
   tgcactggca ccgcggccaa cagccgggac act                                  213
<210> 5
   <211> 71
   <212> PRT
   <213> Artificial Sequence
<220>
   <223> TMB
<400> 5
   Met Gln Lys Glu Arg Phe Asn Ile Asp Met Pro His Arg Phe Lys Val
   1               5                   10                  15
   His Asn Tyr Met Ser Pro Thr Phe Cys Asp His Cys Gly Ser Leu Leu
               20                  25                  30
   Trp Gly Leu Val Lys Gln Gly Leu Lys Cys Glu Asp Cys Gly Met Asn
           35                  40                  45
   Val His His Lys Cys Arg Glu Lys Val Ala Asn Leu Cys Gly Ile Asn
       50                  55                  60
   Gln Lys Leu Leu Ala Glu Ala
   65                  70
<210> 6
   <211> 213
   <212> DNA
   <213> Artificial Sequence
<220>
   <223> TMB
<400> 6
   atgcagaaag aacgcttcaa catcgacatg ccgcaccgct tcaaggttca caactacatg        60
   agccccacct tctgtgacca ctgcggcagc ctgctctggg gactggtgaa gcagggatta       120
   aagtgtgaag actgcggcat gaatgtgcac cataaatgcc gggagaaggt ggccaacctc       180
   tgcggcatca accagaagct tttggctgag gcc                                    213
<210> 7
   <211> 676
   <212> PRT
   <213> Artificial Sequence
<220>
   <223> Protein kinase C mutant, TMD
<400> 7
   Met Ala Pro Phe Leu Arg Ile Ala Phe Asn Ser Tyr Glu Leu Gly Ser
    1               5                  10                  15
   Leu Gln Ala Glu Asp Glu Ala Asn Gln Pro Phe Cys Ala Val Lys Met
               20                  25                  30
   Lys Glu Ala Leu Ser Thr Glu Arg Gly Lys Thr Leu Val Gln Lys Lys
           35                  40                  45
   Pro Thr Met Tyr Pro Glu Trp Lys Ser Thr Phe Asp Ala His Ile Tyr
       50                  55                  60
   Glu Gly Arg Val Ile Gln Ile Val Leu Met Arg Ala Ala Glu Glu Pro
   65                  70                  75                  80
   Val Ser Glu Val Thr Val Gly Val Ser Val Leu Ala Glu Arg Cys Lys
                   85                  90                  95
   Lys Asn Asn Gly Lys Ala Glu Phe Trp Leu Asp Leu Gln Pro Gln Ala
               100                 105                 110
   Lys Val Leu Met Ser Val Gln Tyr Phe Leu Glu Asp Val Asp Cys Lys
           115                 120                 125
   Gln Ser Met Arg Ser Glu Asp Glu Ala Lys Phe Pro Thr Met Asn Arg
       130                 135                 140
   Arg Gly Ala Ile Lys Gln Ala Lys Ile His Tyr Ile Lys Asn His Glu
   145                 150                 155                 160
   Phe Ile Ala Thr Phe Phe Gly Gln Pro Thr Phe Cys Ser Val Cys Lys
                   165                 170                 175
   Asp Phe Val Trp Gly Leu Asn Lys Gln Gly Tyr Lys Cys Arg Gln Cys
               180                 185                 190
   Asn Ala Ala Ile His Lys Lys Cys Ile Asp Lys Ile Ile Gly Arg Cys
           195                 200                 205
   Thr Gly Thr Ala Ala Asn Ser Arg Asp Thr Ile Phe Gln Lys Glu Arg
       210                 215                 220
   Phe Asn Ile Asp Met Pro His Arg Phe Lys Val His Asn Tyr Met Ser
   225                 230                 235                 240
   Pro Thr Phe Cys Asp His Cys Gly Ser Leu Leu Trp Gly Leu Val Lys
                   245                 250                 255
   Gln Gly Leu Lys Cys Glu Asp Cys Gly Met Asn Val His His Lys Cys
               260                 265                 270
   Arg Glu Lys Val Ala Asn Leu Cys Gly Ile Asn Gln Lys Leu Leu Ala
           275                 280                 285
   Glu Ala Leu Asn Gln Val Thr Gln Arg Ala Ser Arg Arg Ser Asp Ser
       290                 295                 300
   Ala Ser Ser Glu Pro Val Gly Ile Phe Gln Gly Phe Glu Lys Lys Thr
   305                 310                 315                 320
   Gly Val Ala Gly Glu Asp Met Gln Asp Asn Ser Gly Thr Tyr Gly Lys
                   325                 330                 335
   Ile Trp Glu Gly Ser Ser Lys Cys Asn Ile Asn Asn Phe Ile Phe His
               340                 345                 350
   Lys Val Leu Gly Lys Gly Ser Phe Gly Lys Val Leu Leu Gly Glu Leu
           355                 360                 365
   Lys Gly Arg Gly Glu Tyr Phe Ala Ile Arg Ala Leu Lys Lys Asp Val
       370                 375                 380
   Val Leu Ile Asp Asp Asp Val Glu Cys Thr Met Val Glu Lys Arg Val
   385                 390                 395                 400
   Leu Thr Leu Ala Ala Glu Asn Pro Phe Leu Thr His Leu Ile Cys Thr
                   405                 410                 415
   Phe Gln Thr Lys Asp His Leu Phe Phe Val Met Glu Phe Leu Asn Gly
   420 425 430
   Gly Asp Leu Met Tyr His Ile Gln Asp Lys Gly Arg Phe Glu Leu Tyr
   435 440 445
   Arg Ala Thr Phe Tyr Ala Ala Glu Ile Met Cys Gly Leu Gln Phe Leu
   450 455 460
   His Ser Lys Gly Ile Ile Tyr Arg Asp Leu Lys Leu Asp Asn Val Leu
   465 470 475 480
   Leu Asp Arg Asp Gly His Ile Lys Ile Ala Asp Phe Gly Met Cys Lys
   485 490 495
   Glu Asn Ile Phe Gly Glu Ser Arg Ala Ser Thr Phe Cys Gly Thr Pro
   500 505 510
   Asp Tyr Ile Ala Pro Glu Ile Leu Gln Gly Leu Lys Tyr Thr Phe Ser
   515 520 525
   Val Asp Trp Trp Ser Phe Gly Val Leu Leu Tyr Glu Met Leu Ile Gly
   530 535 540
   Gln Ser Pro Phe His Gly Asp Asp Glu Asp Glu Leu Phe Glu Ser Ile
   545 550 555 560
   Arg Val Asp Thr Pro His Tyr Pro Arg Trp Ile Thr Lys Glu Ser Lys
   565 570 575
   Asp Ile Leu Glu Lys Leu Phe Glu Arg Glu Pro Thr Lys Arg Leu Gly
   580 585 590
   Val Thr Gly Asn Ile Lys Ile His Pro Phe Phe Lys Thr Ile Asn Trp
   595 600 605
   Thr Leu Leu Glu Lys Arg Arg Leu Glu Pro Pro Phe Arg Pro Lys Val
   610 615 620
   Lys Ser Pro Arg Asp Tyr Ser Asn Phe Asp Gln Glu Phe Leu Asn Glu
   625 630 635 640
   Lys Ala Arg Leu Ser Tyr Ser Asp Lys Asn Leu Ile Asp Ser Met Asp
   645 650 655
   Gln Ser Ala Phe Ala Gly Phe Ser Phe Val Asn Pro Lys Phe Glu His
   660 665 670
   Leu Leu Glu Asp
   675
<210> 8
   <211> 2028
   <212> DNA
   <213> Artificial Sequence
<220>
   <223> Protein kinase C mutant, TMD
<400> 8
   atggcgccgt tcctgcgcat cgccttcaac tcctatgagc tgggctccct gcaggccgag 60
   gacgaggcga accagccctt ctgtgccgtg aagatgaagg aggcgctcag cacagagcgt 120
   gggaaaacac tggtgcagaa gaagccgacc atgtatcctg agtggaagtc gacgttcgac 180
   gcccacatct atgaggggcg cgtcatccag attgtgctaa tgcgggcagc agaggagcca 240
   gtgtctgagg tgaccgtggg tgtgtcggtg ctggccgagc gctgcaagaa gaacaatggc 300
   aaggctgagt tctggctgga cctgcagcct caggccaagg tgttgatgtc tgttcagtat 360
   ttcctggagg acgtggattg caaacagtct atgcgcagtg aggacgaggc caagttccca 420
   acgatgaacc gccgcggagc catcaaacag gccaaaatcc actacatcaa gaaccatgag 480
   tttatcgcca ccttctttgg gcaacccacc ttctgttctg tgtgcaaaga ctttgtctgg 540
   ggcctcaaca agcaaggcta caaatgcagg caatgtaacg ctgccatcca caagaaatgc 600
   atcgacaaga tcatcggcag atgcactggc accgcggcca acagccggga cactatattc 660
   cagaaagaac gcttcaacat cgacatgccg caccgcttca aggttcacaa ctacatgagc 720
   cccaccttct gtgaccactg cggcagcctg ctctggggac tggtgaagca gggattaaag 780
   tgtgaagact gcggcatgaa tgtgcaccat aaatgccggg agaaggtggc caacctctgc 840
   ggcatcaacc agaagctttt ggctgaggcc ttgaaccaag tcacccagag agcctcccgg 900
   agatcagact cagcctcctc agagcctgtt gggatatttc agggtttcga gaagaagacc 960
   ggagttgctg gggaggacat gcaagacaac agtgggacct acggcaagat ctgggagggc 1020
   agcagcaagt gcaacatcaa caacttcatc ttccacaagg tcctgggcaa aggcagcttc 1080
   gggaaggtgc tgcttggaga gctgaagggc agaggagagt actttgccat cagggccctc 1140
   aagaaggatg tggtcctgat cgacgacgac gtggagtgca ccatggttga gaagcgggtg 1200
   ctgacacttg ccgcagagaa tccctttctc acccacctca tctgcacctt ccagaccaag 1260
   gaccacctgt tctttgtgat ggagttcctc aacggggggg acctgatgta ccacatccag 1320
   gacaaaggcc gctttgaact ctaccgtgcc acgttttatg ccgctgagat aatgtgtgga 1380
   ctgcagtttc tacacagcaa gggcatcatt tacagggacc tcaaactgga caatgtgctg 1440
   ttggaccggg atggccacat caagattgcc gactttggga tgtgcaaaga gaacatattc 1500
   ggggagagcc gggccagcac cttctgcggc acccctgact atatcgcccc tgagatccta 1560
   cagggcctga agtacacatt ctctgtggac tggtggtctt tcggggtcct tctgtacgag 1620
   atgctcattg gccagtcccc cttccatggt gatgatgagg atgaactctt cgagtccatc 1680
   cgtgtggaca cgccacatta tccccgctgg atcaccaagg agtccaagga catcctggag 1740
   aagctctttg aaagggaacc aaccaagagg ctgggagtga cgggaaacat caaaatccac    1800
   cccttcttca agaccataaa ctggactctg ctggaaaagc ggaggttgga gccacccttc    1860
   aggcccaaag tgaagtcacc cagagactac agtaactttg accaggagtt cctgaacgag    1920
   aaggcgcgcc tctcctacag cgacaagaac ctcatcgact ccatggacca gtctgcattc    1980
   gctggcttct cctttgtgaa ccccaaattc gagcacctcc tggaagat                 2028
<210> 9
   <211> 239
   <212> PRT
   <213> Artificial Sequence
<220>
   <223> EGFP
<400> 9
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
                   100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 10
   <211> 716
   <212> DNA
   <213> Artificial Sequence
<220>
   <223> EGFP
<400> 10
   tggtgagcaa gggcgaggag ctgttcaccg gggtggtgcc catcctggtc gagctggacg      60
   gcgacgtaaa cggccacaag ttcagcgtgt ccggcgaggg cgagggcgat gccacctacg     120
   gcaagctgac cctgaagttc atctgcacca ccggcaagct gcccgtgccc tggcccaccc     180
   tcgtgaccac cctgacctac ggcgtgcagt gcttcagccg ctaccccgac cacatgaagc     240
   agcacgactt cttcaagtcc gccatgcccg aaggctacgt ccaggagcgc accatcttct     300
   tcaaggacga cggcaactac aagacccgcg ccgaggtgaa gttcgagggc gacaccctgg     360
   tgaaccgcat cgagctgaag ggcatcgact tcaaggagga cggcaacatc ctggggcaca     420
   agctggagta caactacaac agccacaacg tctatatcat ggccgacaag cagaagaacg     480
   gcatcaaggt gaacttcaag atccgccaca acatcgagga cggcagcgtg cagctcgccg     540
   accactacca gcagaacacc cccatcggcg acggccccgt gctgctgccc gacaaccact     600
   acctgagcac ccagtccgcc ctgagcaaag accccaacga gaagcgcgat cacatggtcc     660
   tgctggagtt cgtgaccgcc gccgggatca ctctcggcat ggacgagctg tacaag         716
<210> 11
   <211> 225
   <212> PRT
   <213> Artificial Sequence
<220>
   <223> mRFP
<400> 11
   Met Ala Ser Ser Glu Asp Val Ile Lys Glu Phe Met Arg Phe Lys Val
   1               5                   10                  15
   Arg Met Glu Gly Ser Val Asn Gly His Glu Phe Glu Ile Glu Gly Glu
               20                  25                  30
   Gly Glu Gly Arg Pro Tyr Glu Gly Thr Gln Thr Ala Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Ala Tyr Val Lys His Pro Ala Asp Ile Pro
   65                  70                  75                  80
   Asp Tyr Leu Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Glu Phe Ile Tyr Lys Val Lys Leu Arg Gly Thr Asn
           115                 120                 125
   Phe Pro Ser Asp Gly Pro Val Met Gln Lys Lys Thr Met Gly Trp Glu
       130                 135                 140
   Ala Ser Thr Glu Arg Met Tyr Pro Glu Asp Gly Ala Leu Lys Gly Glu
   145                 150                 155                 160
   Ile Lys Met Arg Leu Lys Leu Lys Asp Gly Gly His Tyr Asp Ala Glu
                   165                 170                 175
   Val Lys Thr Thr Tyr Met Ala Lys Lys Pro Val Gln Leu Pro Gly Ala
               180                 185                 190
   Tyr Lys Thr Asp Ile Lys Leu Asp Ile Thr Ser His Asn Glu Asp Tyr
           195                 200                 205
   Thr Ile Val Glu Gln Tyr Glu Arg Ala Glu Gly Arg His Ser Thr Gly
       210                 215                 220
   Ala
   225
<210> 12
   <211> 674
   <212> DNA
   <213> Artificial Sequence
<220>
   <223> mRFP
<400> 12
   tggcctcctc cgaggacgtc atcaaggagt tcatgcgctt caaggtgcgc atggagggct      60
   ccgtgaacgg ccacgagttc gagatcgagg gcgagggcga gggccgcccc tacgagggca     120
   cccagaccgc caagctgaag gtgaccaagg gcggccccct gcccttcgcc tgggacatcc     180
   tgtcccctca gttccagtac ggctccaagg cctacgtgaa gcaccccgcc gacatccccg     240
   actacttgaa gctgtccttc cccgagggct tcaagtggga gcgcgtgatg aacttcgagg     300
   acggcggcgt ggtgaccgtg acccaggact cctccctgca ggacggcgag ttcatctaca     360
   aggtgaagct gcgcggcacc aacttcccct ccgacggccc cgtaatgcag aagaagacca     420
   tgggctggga ggcctccacc gagcggatgt accccgagga cggcgccctg aagggcgaga     480
   tcaagatgag gctgaagctg aaggacggcg gccactacga cgccgaggtc aagaccacct     540
   acatggccaa gaagcccgtg cagctgcccg gcgcctacaa gaccgacatc aagctggaca     600
   tcacctccca caacgaggac tacaccatcg tggaacagta cgagcgcgcc gagggccgcc     660
   actccaccgg cgcc                                                       674
<210> 13
   <211> 638
   <212> DNA
   <213> Artificial Sequence
<220>
   <223> Streptomyces avidinii gene for streptavidin
<400> 13
   ccctccgtcc ccgccgggca acaactaggg agtatttttc gtgtctcaca tgcgcaagat      60
   cgtcgttgca gccatcgccg tttccctgac cacggtctcg attacggcca gcgcttcggc     120
   agacccctcc aaggactcga aggcccaggt ctcggccgcc gaggccggca tcaccggcac     180
   ctggtacaac cagctcggct cgaccttcat cgtgaccgcg ggcgccgacg gcgccctgac     240
   cggaacctac gagtcggccg tcggcaacgc cgagagccgc tacgtcctga ccggtcgtta     300
   cgacagcgcc ccggccaccg acggcagcgg caccgccctc ggttggacgg tggcctggaa     360
   gaataactac cgcaacgccc actccgcgac cacgtggagc ggccagtacg tcggcggcgc     420
   cgaggcgagg atcaacaccc agtggctgct gacctccggc accaccgagg ccaacgcctg     480
   gaagtccacg ctggtcggcc acgacacctt caccaaggtg aagccgtccg ccgcctccat     540
   cgacgcggcg aagaaggccg gcgtcaacaa cggcaacccg ctcgacgccg ttcagcagta     600
   gtcgcgtccc ggcaccggcg ggtgccggga cctcggcc                            638
<210> 14
   <211> 4685
   <212> DNA
   <213> Artificial Sequence
<220>
   <223> pmRFP-C3 vector
<400> 14
   tagttattaa tagtaatcaa ttacggggtc attagttcat agcccatata tggagttccg      60
   cgttacataa cttacggtaa atggcccgcc tggctgaccg cccaacgacc cccgcccatt     120
   gacgtcaata atgacgtatg ttcccatagt aacgccaata gggactttcc attgacgtca      180
   atgggtggag tatttacggt aaactgccca cttggcagta catcaagtgt atcatatgcc      240
   aagtacgccc cctattgacg tcaatgacgg taaatggccc gcctggcatt atgcccagta      300
   catgacctta tgggactttc ctacttggca gtacatctac gtattagtca tcgctattac      360
   catggtgatg cggttttggc agtacatcaa tgggcgtgga tagcggtttg actcacgggg      420
   atttccaagt ctccacccca ttgacgtcaa tgggagtttg ttttggcacc aaaatcaacg      480
   ggactttcca aaatgtcgta acaactccgc cccattgacg caaatgggcg gtaggcgtgt      540
   acggtgggag gtctatataa gcagagctgg tttagtgaac cgtcagatcc gctagcgcta      600
   ccggtcgcca ccatggcctc ctccgaggac gtcatcaagg agttcatgcg cttcaaggtg      660
   cgcatggagg gctccgtgaa cggccacgag ttcgagatcg agggcgaggg cgagggccgc      720
   ccctacgagg gcacccagac cgccaagctg aaggtgacca agggcggccc cctgcccttc      780
   gcctgggaca tcctgtcccc tcagttccag tacggctcca aggcctacgt gaagcacccc      840
   gccgacatcc ccgactactt gaagctgtcc ttccccgagg gcttcaagtg ggagcgcgtg      900
   atgaacttcg aggacggcgg cgtggtgacc gtgacccagg actcctccct gcaggacggc      960
   gagttcatct acaaggtgaa gctgcgcggc accaacttcc cctccgacgg ccccgtaatg     1020
   cagaagaaga ccatgggctg ggaggcctcc accgagcgga tgtaccccga ggacggcgcc     1080
   ctgaagggcg agatcaagat gaggctgaag ctgaaggacg gcggccacta cgacgccgag     1140
   gtcaagacca cctacatggc caagaagccc gtgcagctgc ccggcgccta caagaccgac     1200
   atcaagctgg acatcacctc ccacaacgag gactacacca tcgtggaaca gtacgagcgc     1260
   gccgagggcc gccactccac cggcgccctg tcagatctcg agctcaagct tcgaattctg     1320
   cagtcgacgg taccgcgggc ccgggatcca ccggatctag ataactgatc ataatcagcc    1380
   ataccacatt tgtagaggtt ttacttgctt taaaaaacct cccacacctc cccctgaacc    1440
   tgaaacataa aatgaatgca attgttgttg ttaacttgtt tattgcagct tataatggtt    1500
   acaaataaag caatagcatc acaaatttca caaataaagc atttttttca ctgcattcta    1560
   gttgtggttt gtccaaactc atcaatgtat cttaacgcgt aaattgtaag cgttaatatt    1620
   ttgttaaaat tcgcgttaaa tttttgttaa atcagctcat tttttaacca ataggccgaa    1680
   atcggcaaaa tcccttataa atcaaaagaa tagaccgaga tagggttgag tgttgttcca    1740
   gtttggaaca agagtccact attaaagaac gtggactcca acgtcaaagg gcgaaaaacc    1800
   gtctatcagg gcgatggccc actacgtgaa ccatcaccct aatcaagttt tttggggtcg    1860
   aggtgccgta aagcactaaa tcggaaccct aaagggagcc cccgatttag agcttgacgg    1920
   ggaaagccgg cgaacgtggc gagaaaggaa gggaagaaag cgaaaggagc gggcgctagg    1980
   gcgctggcaa gtgtagcggt cacgctgcgc gtaaccacca cacccgccgc gcttaatgcg    2040
   ccgctacagg gcgcgtcagg tggcactttt cggggaaatg tgcgcggaac ccctatttgt    2100
   ttatttttct aaatacattc aaatatgtat ccgctcatga gacaataacc ctgataaatg    2160
   cttcaataat attgaaaaag gaagagtcct gaggcggaaa gaaccagctg tggaatgtgt    2220
   gtcagttagg gtgtggaaag tccccaggct ccccagcagg cagaagtatg caaagcatgc    2280
   atctcaatta gtcagcaacc aggtgtggaa agtccccagg ctccccagca ggcagaagta    2340
   tgcaaagcat gcatctcaat tagtcagcaa ccatagtccc gcccctaact ccgcccatcc    2400
   cgcccctaac tccgcccagt tccgcccatt ctccgcccca tggctgacta atttttttta    2460
   tttatgcaga ggccgaggcc gcctcggcct ctgagctatt ccagaagtag tgaggaggct    2520
   tttttggagg cctaggcttt tgcaaagatc gatcaagaga caggatgagg atcgtttcgc 2580
   atgattgaac aagatggatt gcacgcaggt tctccggccg cttgggtgga gaggctattc 2640
   ggctatgact gggcacaaca gacaatcggc tgctctgatg ccgccgtgtt ccggctgtca 2700
   gcgcaggggc gcccggttct ttttgtcaag accgacctgt ccggtgccct gaatgaactg 2760
   caagacgagg cagcgcggct atcgtggctg gccacgacgg gcgttccttg cgcagctgtg 2820
   ctcgacgttg tcactgaagc gggaagggac tggctgctat tgggcgaagt gccggggcag 2880
   gatctcctgt catctcacct tgctcctgcc gagaaagtat ccatcatggc tgatgcaatg 2940
   cggcggctgc atacgcttga tccggctacc tgcccattcg accaccaagc gaaacatcgc 3000
   atcgagcgag cacgtactcg gatggaagcc ggtcttgtcg atcaggatga tctggacgaa 3060
   gagcatcagg ggctcgcgcc agccgaactg ttcgccaggc tcaaggcgag catgcccgac 3120
   ggcgaggatc tcgtcgtgac ccatggcgat gcctgcttgc cgaatatcat ggtggaaaat 3180
   ggccgctttt ctggattcat cgactgtggc cggctgggtg tggcggaccg ctatcaggac 3240
   atagcgttgg ctacccgtga tattgctgaa gagcttggcg gcgaatgggc tgaccgcttc 3300
   ctcgtgcttt acggtatcgc cgctcccgat tcgcagcgca tcgccttcta tcgccttctt 3360
   gacgagttct tctgagcggg actctggggt tcgaaatgac cgaccaagcg acgcccaacc 3420
   tgccatcacg agatttcgat tccaccgccg ccttctatga aaggttgggc ttcggaatcg 3480
   ttttccggga cgccggctgg atgatcctcc agcgcgggga tctcatgctg gagttcttcg 3540
   cccaccctag ggggaggcta actgaaacac ggaaggagac aataccggaa ggaacccgcg 3600
   ctatgacggc aataaaaaga cagaataaaa cgcacggtgt tgggtcgttt gttcataaac 3660
   gcggggttcg gtcccagggc tggcactctg tcgatacccc accgagaccc cattggggcc 3720
   aatacgcccg cgtttcttcc ttttccccac cccacccccc aagttcgggt gaaggcccag     3780
   ggctcgcagc caacgtcggg gcggcaggcc ctgccatagc ctcaggttac tcatatatac     3840
   tttagattga tttaaaactt catttttaat ttaaaaggat ctaggtgaag atcctttttg     3900
   ataatctcat gaccaaaatc ccttaacgtg agttttcgtt ccactgagcg tcagaccccg     3960
   tagaaaagat caaaggatct tcttgagatc ctttttttct gcgcgtaatc tgctgcttgc     4020
   aaacaaaaaa accaccgcta ccagcggtgg tttgtttgcc ggatcaagag ctaccaactc     4080
   tttttccgaa ggtaactggc ttcagcagag cgcagatacc aaatactgtc cttctagtgt     4140
   agccgtagtt aggccaccac ttcaagaact ctgtagcacc gcctacatac ctcgctctgc     4200
   taatcctgtt accagtggct gctgccagtg gcgataagtc gtgtcttacc gggttggact     4260
   caagacgata gttaccggat aaggcgcagc ggtcgggctg aacggggggt tcgtgcacac     4320
   agcccagctt ggagcgaacg acctacaccg aactgagata cctacagcgt gagctatgag     4380
   aaagcgccac gcttcccgaa gggagaaagg cggacaggta tccggtaagc ggcagggtcg     4440
   gaacaggaga gcgcacgagg gagcttccag ggggaaacgc ctggtatctt tatagtcctg     4500
   tcgggtttcg ccacctctga cttgagcgtc gatttttgtg atgctcgtca ggggggcgga     4560
   gcctatggaa aaacgccagc aacgcggcct ttttacggtt cctggccttt tgctggcctt     4620
   ttgctcacat gttctttcct gcgttatccc ctgattctgt ggataaccgt attaccgcca     4680
   tgcat                                                                 4685
<210> 15
   <211> 4727
   <212> DNA
   <213> Artificial Sequence
<220>
   <223> pEGFP-C3 vector
<400> 15
   tagttattaa tagtaatcaa ttacggggtc attagttcat agcccatata tggagttccg      60
   cgttacataa cttacggtaa atggcccgcc tggctgaccg cccaacgacc cccgcccatt     120
   gacgtcaata atgacgtatg ttcccatagt aacgccaata gggactttcc attgacgtca     180
   atgggtggag tatttacggt aaactgccca cttggcagta catcaagtgt atcatatgcc     240
   aagtacgccc cctattgacg tcaatgacgg taaatggccc gcctggcatt atgcccagta     300
   catgacctta tgggactttc ctacttggca gtacatctac gtattagtca tcgctattac     360
   catggtgatg cggttttggc agtacatcaa tgggcgtgga tagcggtttg actcacgggg     420
   atttccaagt ctccacccca ttgacgtcaa tgggagtttg ttttggcacc aaaatcaacg     480
   ggactttcca aaatgtcgta acaactccgc cccattgacg caaatgggcg gtaggcgtgt     540
   acggtgggag gtctatataa gcagagctgg tttagtgaac cgtcagatcc gctagcgcta     600
   ccggtcgcca ccatggtgag caagggcgag gagctgttca ccggggtggt gcccatcctg     660
   gtcgagctgg acggcgacgt aaacggccac aagttcagcg tgtccggcga gggcgagggc     720
   gatgccacct acggcaagct gaccctgaag ttcatctgca ccaccggcaa gctgcccgtg     780
   ccctggccca ccctcgtgac caccctgacc tacggcgtgc agtgcttcag ccgctacccc     840
   gaccacatga agcagcacga cttcttcaag tccgccatgc ccgaaggcta cgtccaggag     900
   cgcaccatct tcttcaagga cgacggcaac tacaagaccc gcgccgaggt gaagttcgag     960
   ggcgacaccc tggtgaaccg catcgagctg aagggcatcg acttcaagga ggacggcaac    1020
   atcctggggc acaagctgga gtacaactac aacagccaca acgtctatat catggccgac    1080
   aagcagaaga acggcatcaa ggtgaacttc aagatccgcc acaacatcga ggacggcagc 1140
   gtgcagctcg ccgaccacta ccagcagaac acccccatcg gcgacggccc cgtgctgctg 1200
   cccgacaacc actacctgag cacccagtcc gccctgagca aagaccccaa cgagaagcgc 1260
   gatcacatgg tcctgctgga gttcgtgacc gccgccggga tcactctcgg catggacgag 1320
   ctgtacaagt actcagatct cgagctcaag cttcgaattc tgcagtcgac ggtaccgcgg 1380
   gcccgggatc caccggatct agataactga tcataatcag ccataccaca tttgtagagg 1440
   ttttacttgc tttaaaaaac ctcccacacc tccccctgaa cctgaaacat aaaatgaatg 1500
   caattgttgt tgttaacttg tttattgcag cttataatgg ttacaaataa agcaatagca 1560
   tcacaaattt cacaaataaa gcattttttt cactgcattc tagttgtggt ttgtccaaac 1620
   tcatcaatgt atcttaacgc gtaaattgta agcgttaata ttttgttaaa attcgcgtta 1680
   aatttttgtt aaatcagctc attttttaac caataggccg aaatcggcaa aatcccttat 1740
   aaatcaaaag aatagaccga gatagggttg agtgttgttc cagtttggaa caagagtcca 1800
   ctattaaaga acgtggactc caacgtcaaa gggcgaaaaa ccgtctatca gggcgatggc 1860
   ccactacgtg aaccatcacc ctaatcaagt tttttggggt cgaggtgccg taaagcacta 1920
   aatcggaacc ctaaagggag cccccgattt agagcttgac ggggaaagcc ggcgaacgtg 1980
   gcgagaaagg aagggaagaa agcgaaagga gcgggcgcta gggcgctggc aagtgtagcg 2040
   gtcacgctgc gcgtaaccac cacacccgcc gcgcttaatg cgccgctaca gggcgcgtca 2100
   ggtggcactt ttcggggaaa tgtgcgcgga acccctattt gtttattttt ctaaatacat 2160
   tcaaatatgt atccgctcat gagacaataa ccctgataaa tgcttcaata atattgaaaa 2220
   aggaagagtc ctgaggcgga aagaaccagc tgtggaatgt gtgtcagtta gggtgtggaa 2280
   agtccccagg ctccccagca ggcagaagta tgcaaagcat gcatctcaat tagtcagcaa 2340
   ccaggtgtgg aaagtcccca ggctccccag caggcagaag tatgcaaagc atgcatctca 2400
   attagtcagc aaccatagtc ccgcccctaa ctccgcccat cccgccccta actccgccca 2460
   gttccgccca ttctccgccc catggctgac taattttttt tatttatgca gaggccgagg 2520
   ccgcctcggc ctctgagcta ttccagaagt agtgaggagg cttttttgga ggcctaggct 2580
   tttgcaaaga tcgatcaaga gacaggatga ggatcgtttc gcatgattga acaagatgga 2640
   ttgcacgcag gttctccggc cgcttgggtg gagaggctat tcggctatga ctgggcacaa 2700
   cagacaatcg gctgctctga tgccgccgtg ttccggctgt cagcgcaggg gcgcccggtt 2760
   ctttttgtca agaccgacct gtccggtgcc ctgaatgaac tgcaagacga ggcagcgcgg 2820
   ctatcgtggc tggccacgac gggcgttcct tgcgcagctg tgctcgacgt tgtcactgaa 2880
   gcgggaaggg actggctgct attgggcgaa gtgccggggc aggatctcct gtcatctcac 2940
   cttgctcctg ccgagaaagt atccatcatg gctgatgcaa tgcggcggct gcatacgctt 3000
   gatccggcta cctgcccatt cgaccaccaa gcgaaacatc gcatcgagcg agcacgtact 3060
   cggatggaag ccggtcttgt cgatcaggat gatctggacg aagagcatca ggggctcgcg 3120
   ccagccgaac tgttcgccag gctcaaggcg agcatgcccg acggcgagga tctcgtcgtg 3180
   acccatggcg atgcctgctt gccgaatatc atggtggaaa atggccgctt ttctggattc 3240
   atcgactgtg gccggctggg tgtggcggac cgctatcagg acatagcgtt ggctacccgt 3300
   gatattgctg aagagcttgg cggcgaatgg gctgaccgct tcctcgtgct ttacggtatc 3360
   gccgctcccg attcgcagcg catcgccttc tatcgccttc ttgacgagtt cttctgagcg 3420
   ggactctggg gttcgaaatg accgaccaag cgacgcccaa cctgccatca cgagatttcg 3480
   attccaccgc cgccttctat gaaaggttgg gcttcggaat cgttttccgg gacgccggct 3540
   ggatgatcct ccagcgcggg gatctcatgc tggagttctt cgcccaccct agggggaggc 3600
   taactgaaac acggaaggag acaataccgg aaggaacccg cgctatgacg gcaataaaaa 3660
   gacagaataa aacgcacggt gttgggtcgt ttgttcataa acgcggggtt cggtcccagg 3720
   gctggcactc tgtcgatacc ccaccgagac cccattgggg ccaatacgcc cgcgtttctt 3780
   ccttttcccc accccacccc ccaagttcgg gtgaaggccc agggctcgca gccaacgtcg 3840
   gggcggcagg ccctgccata gcctcaggtt actcatatat actttagatt gatttaaaac 3900
   ttcattttta atttaaaagg atctaggtga agatcctttt tgataatctc atgaccaaaa 3960
   tcccttaacg tgagttttcg ttccactgag cgtcagaccc cgtagaaaag atcaaaggat 4020
   cttcttgaga tccttttttt ctgcgcgtaa tctgctgctt gcaaacaaaa aaaccaccgc 4080
   taccagcggt ggtttgtttg ccggatcaag agctaccaac tctttttccg aaggtaactg 4140
   gcttcagcag agcgcagata ccaaatactg tccttctagt gtagccgtag ttaggccacc 4200
   acttcaagaa ctctgtagca ccgcctacat acctcgctct gctaatcctg ttaccagtgg 4260
   ctgctgccag tggcgataag tcgtgtctta ccgggttgga ctcaagacga tagttaccgg 4320
   ataaggcgca gcggtcgggc tgaacggggg gttcgtgcac acagcccagc ttggagcgaa 4380
   cgacctacac cgaactgaga tacctacagc gtgagctatg agaaagcgcc acgcttcccg 4440
   aagggagaaa ggcggacagg tatccggtaa gcggcagggt cggaacagga gagcgcacga 4500
   gggagcttcc agggggaaac gcctggtatc tttatagtcc tgtcgggttt cgccacctct 4560
   gacttgagcg tcgatttttg tgatgctcgt caggggggcg gagcctatgg aaaaacgcca 4620
   gcaacgcggc ctttttacgg ttcctggcct tttgctggcc ttttgctcac atgttctttc 4680
   ctgcgttatc ccctgattct gtggataacc gtattaccgc catgcat 4727
<210> 16
   <211> 39
   <212> DNA
   <213> Artificial Sequence
<220>
   <223> AzG-F
<400> 16
   ggcaccggtc gccaccatgg accccatggt gagtgtgat 39
<210> 17
   <211> 35
   <212> DNA
   <213> Artificial Sequence
<220>
   <223> AzG-R
<400> 17
   ggcagatctg acagcttggc ctgactcggc agcat 35
<210> 18
   <211> 24
   <212> DNA
   <213> Artificial Sequence
<220>
   <223> HcR-F
<400> 18
   gccaccggtc gccaccatgg tgag 24
<210> 19
   <211> 38
   <212> DNA
   <213> Artificial Sequence
<220>
   <223> HcR-R
<400> 19
   gccgcggccg cttatcagtt ggccttctcg ggcaggtc 38